# EUROPÄISCHE PATENTSCHRIFT

(11) **EP 2 282 991 B1**
(45) Veröffentlichungstag und Bekanntmachung des Hinweises auf die Patenterteilung: **24.01.2018**
(21) Anmeldenummer: 09737876.4
(22) Anmeldetag: 29.04.2009
(51) Int. Cl.: C07C 315/02, C07C 315/04, C07C 317/18, C07C 317/24, C07C 319/14, C07C 319/20, C07C 323/12, C07C 323/22, C07C 327/32, C07D 337/08, C07D 409/12, C07D 453/04

(54) **VERFAHREN ZUR HERSTELLUNG VON 1,4-BENZOTHIEPIN-1,1-DIOXIDDERIVATEN**
METHOD FOR THE PRODUCTION OF 1,4-BENZOTHIEPIN-1,1-DIOXIDE DERIVATIVES
PROCÉDÉ DE PRÉPARATION DE DÉRIVÉS DE 1,4-BENZOTHIÉPINE-1,1-DIOXYDE

(30) Priorität: 02.05.2008 DE 102008022017; 07.02.2009 DE 102009007825; 24.03.2009 DE 102009014637
(43) Veröffentlichungstag der Anmeldung: 16.02.2011
(73) Patentinhaber: Sanofi-Aventis Deutschland GmbH, 65929 Frankfurt am Main (DE)
(72) Erfinder: BILLEN, Guenter, 65926 Frankfurt am Main (DE); FRICK, Wendelin, 65926 Frankfurt am Main (DE); LARKIN, John, Patrick, Leicestershire LE16 7 (GB); LEMAITRE, Guy, F-77280 Othis (FR); BENDETTI, Françoise, F-75013 Paris (FR); BOFFELLI, Philippe, F-75013 Paris (FR); GODARD, Jean-Yves, F-75013 Paris (FR); MASSON, Christian, F-75013 Paris (FR); CROCQ, Véronique, F-75013 Paris (FR); LAFONT, Sylvaine, F-75013 Paris (FR); HULSHOF, Jos, NL-9747 AT Groningen (NL)
(74) Vertreter: Weickmann & Weickmann PartmbB
(86) Internationale Anmeldenummer: PCT/EP2009/003102
(87) Internationale Veröffentlichungsnummer: WO 2009/132832

(56) Entgegenhaltungen:
- EP-B1- 2 084 172
- US-A- 5 994 391
- US-A1- 2004 147 774

## Beschreibung

Die Erfindung betrifft ein Verfahren zur Herstellung von 1,4-Benzothiepin-1,1-Dioxidderivaten, die mit Benzylresten substituiert sind.

Es sind bereits 1,4-Benzothiepin-1,1-Dioxidderivate beschrieben worden (US 5,994,391). Das in US 5,994,391 beschriebene Verfahren führt allerdings zu Racematen. Die Synthese der optisch reinen Verbindungen (Zwischenprodukte oder Endprodukte) erfordert aufwendige chromatographische Reinigungsschritte. Siehe zum Beispiel Position 3 der Verbindung I aus US 5,994,391 oder auch die Zwischenprodukte LI oder XLI aus US 5,994,391.

Der Erfindung lag die Aufgabe zugrunde, ein verbessertes Verfahren zur Herstellung von bestimmten entantiomerenreinen 1,4-Benzothiepin-1,1-Dioxidderivaten zur Verfügung zu stellen. Insbesondere sollten die Stereozentren an Position 3, 4 und 5 des Thiepinsystems der Verbindung der Formel I optisch rein aufgebaut bzw. erhalten werden.

Die Erfindung betrifft daher ein verbessertes Verfahren zur Herstellung der Verbindungen der Formel I 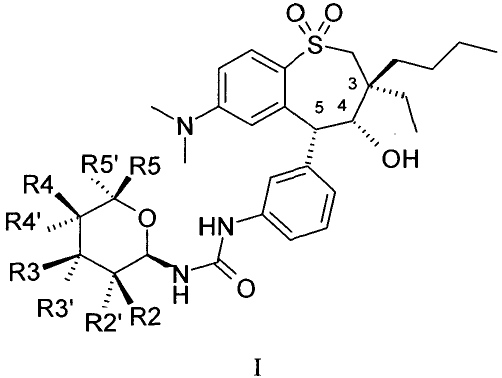 worin bedeuten
R2, R2', R3, R3', R4, R4', R5, R5' unabhängig voneinander H, Cl, Br, I, OH, -(CH₂)-OH, CF₃, NO₂ N₃, CN, S(O)ₚ-R6, O-S(O)ₚ-R6, (C₁-C₆)-Alkylen-S(O)ₚ-R6, (C₁-C₆)-Alkylen-O-S(O)ₚ-R6, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
   Phenyl, -(CH₂)-Phenyl, -(CH₂)ₙ-Phenyl, O-Phenyl, O-(CH₂)ₘ-Phenyl, -(CH₂)-O-(CH₂)ₘ-Phenyl, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
wobei immer mindestens einer der Reste R2, R2', R3, R3', R4, R4', R5, R5' die Bedeutung -O-(CH₂)ₘ-Phenyl oder -(CH₂)-O-(CH₂)ₘ-Phenyl besitzt, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R6 H, OH, (C₁-C₆)-Alkyl, NH₂, NH(C₁-C₆)-Alkyl, N((C₁-C₆)-Alkyl)₂;
n 2, 3, 4, 5, 6;
m 1,2,3,4,5,6;
p 0, 1, 2;
sowie deren pharmazeutisch verträgliche Salze.

Die Erfindung betrifft weiterhin verbesserte Verfahren zur Herstellung der Verbindungen der Formeln Ia, Ib und Ic 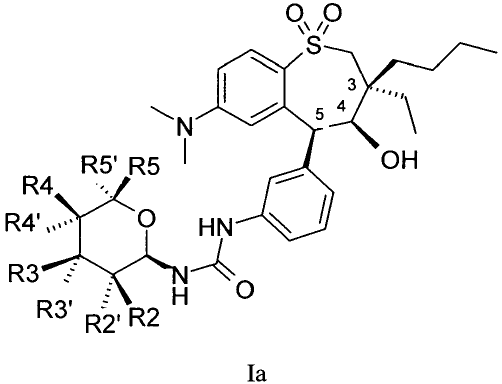 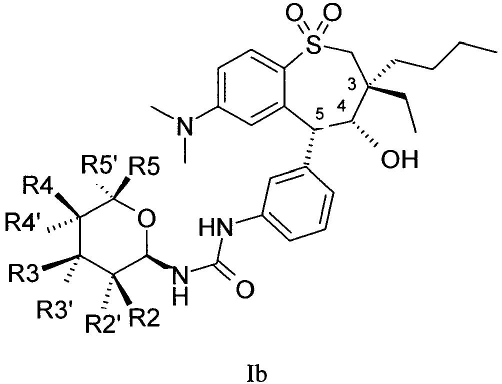 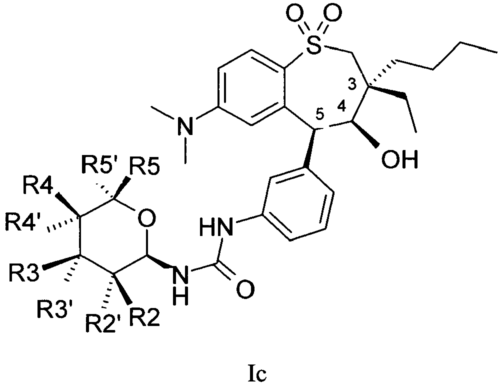 worin bedeuten
R2, R2', R3, R3', R4, R4', R5, R5' unabhängig voneinander H, Cl, Br, I, OH, -(CH₂)-OH, CF₃, NO₂ N₃, CN, S(O)ₚ-R6, O-S(O)ₚ-R6, (C₁-C₆)-Alkylen-S(O)ₚ-R6, (C₁-C₆)-Alkylen-O-S(O)ₚ-R6, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
   Phenyl, -(CH₂)-Phenyl, -(CH₂)ₙ-Phenyl, O-Phenyl, O-(CH₂)ₘ-Phenyl, -(CH₂)-O-(CH₂)ₘ-Phenyl, wobei der Phenylring ein bis 3-fach substituiert sein kann mit F, Cl, Br, I, OH, CF₃, NO₂ CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
wobei immer mindestens einer der Reste R2, R2', R3, R3', R4, R4', R5, R5' die Bedeutung -O-(CH₂)ₘ-Phenyl oder -(CH₂)-O-(CH₂)ₘ-Phenyl besitzt, wobei der Phenylring ein bis 3-fach substituiert sein kann mit F, Cl, Br, I, OH, CF₃, NO₂ CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R6 H, OH, (C₁-C₆)-Alkyl, NH₂, NH(C₁-C₆)-Alkyl, N((C₁-C₆)-Alkyl)₂;
n 2, 3, 4, 5, 6;
m 1,2,3,4,5,6;
p 0, 1, 2;
sowie deren pharmazeutisch verträgliche Salze.

Können Reste oder Substituenten mehrfach in den Verbindungen der Formeln I auftreten, so können sie alle unabhängig voneinander die angegebene Bedeutung haben und gleich oder verschieden sein.

Die Alkyl-, Alkenyl-, Alkinyl-, Alkylen-, Alkenylen- und Alkinylenreste in den Resten R, R1, R2, R2', R3, R3', R4, R4', R5, R5' und R6 können sowohl geradkettig wie verzweigt sein. Eine Ausführungsform der Erfindung bezieht sich weiterhin auch auf einzelne Reaktionsschritte sowie Zwischenprodukte dieses Verfahrens zur Herstellung der Verbindungen der Formeln 10 und 10a, das folgende Schritte aufweist:
Die Verbindung der Formel 10 oder 10a lässt sich beispielsweise herstellen indem man die Verbindung der Formel 8 mit einem Thiol der Formel 5 oder 5a, in Gegenwart einer geeigneten Base, wie zum Beispiel Natriumcarbonat, Kaliumcarbonat oderCaesiumcarbonat in einem geeigneten Lösungsmittel wie zum Beispiel Toluol, Dimethylformamid, Dimethylsulfoxid oder N-Methylpyrrolidon umsetzt.
Die Reaktionstemperatur beträgt dabei von 20 °C bis 120 °C, bevorzugt von 40 °C bis 80 °C. Die Reaktionszeit liegt im Allgemeinen bei 0,5 bis 8 Stunden, je nach Zusammensetzung des Gemisches und des gewählten Temperaturbereiches.
Die erhaltene Verbindung der Formel 10 oder 10a wird anschließend durch wässrige Aufarbeitung und Extraktion mit einem geeigneten Lösungsmittel, beispielsweise Toluol, Ethylacetat oder Dichlormethan aus dem Reaktionsgemisch abgetrennt.

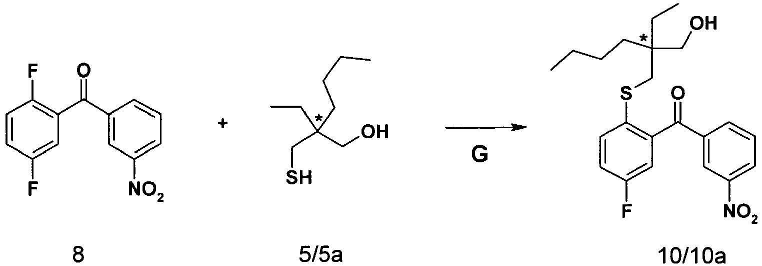

Der Stern an einem Kohlenstoffatom in der Verbindung der Formel 10/10a bedeutet, dass das jeweilige Kohlenstoffatom chiral ist und die Verbindung entweder als R- oder S-Enantiomer oder als Gemisch der beiden Enantiomeren vorliegt.

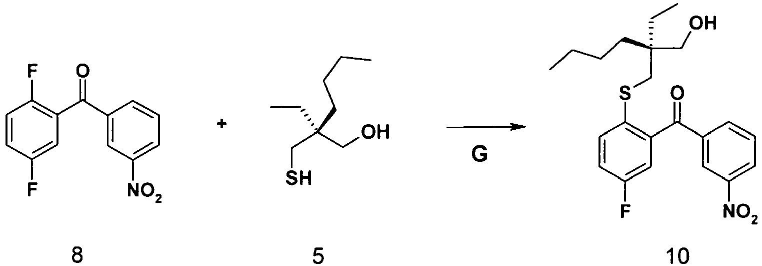

Bevorzugt wird die Verbindung 10 oder. 10a in enantiomerenreiner Form wie zum Beispiel durch Umsetzung der Verbindung der Formel 8 mit der Verbindung der Formel 5 unter den angegebenen Reaktionsbedingungen hergestellt. Die Herstellung der Verbindung der Formel 10a gelingt analog durch Umsetzung mit der Verbindung der Formel 5a.

Die Verbindung der Formel 8 lässt sich beispielsweise herstellen indem man die Verbindung der Formel 7 mit 3-Nitrobenzoylchlorid, in Gegenwart eines geeigneten Katalysators, wie zum Beispiel Aluminium-(III)-chlorid, umsetzt.
Die Reaktionstemperatur beträgt dabei von 40 °C bis 140 °C, bevorzugt von 80 °C bis 120 °C. Die Reaktionszeit liegt im allgemeinen bei 2 bis 24 Stunden, je nach Zusammensetzung des Gemisches und des gewählten Temperaturbereiches.
Die erhaltene Verbindung der Formel 8 wird anschließend durch wässrige Aufarbeitung und Extraktion mit einem geeigneten Lösungsmittel, beispielsweise Ethylacetat oder Dichlormethan und anschließende Kristallisation aus dem Reaktionsgemisch abgetrennt. 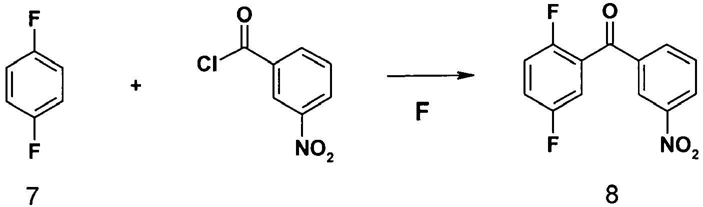 Die Verbindung der Formel 1 lässt sich beispielsweise herstellen indem man 2-butyl-2-ethyl-1,3-propandiol mit geeigneten Oxidationsmitteln, wie zum Beispiel Kaliumpermanganat, umsetzt.
Die Reaktionstemperatur beträgt dabei von 0 °C bis 100°C, bevorzugt von 0 °C bis 40 °C. Die Reaktionszeit liegt im allgemeinen bei 2 bis 8 Stunden, je nach Zusammensetzung des Gemisches und des gewählten Temperaturbereiches. Die erhaltene Verbindung der Formel 1 wird anschließend durch wässrige Aufarbeitung und Extraktion mit einem geeigneten Lösungsmittel, beispielsweise Ethylacetat oder Dichlormethan aus dem Reaktionsgemisch abgetrennt. Eine Reinigung kann mit Hilfe einer Vakuumdestillation durchgeführt werden. Weiterhin kann die Verbindung der Formel 1 nach den literaturbekannten Verfahren gewonnen werden. 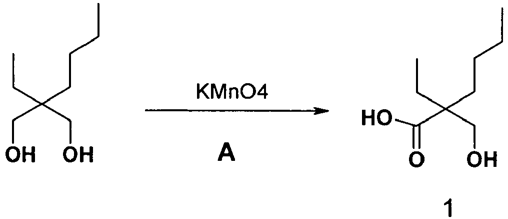 Boehm, Andreas; Petersen, Hermann; Stohrer, Juergen. Regioselective hydroxymethylation process for the preparation of α,α-dialkyl-α-hydroxymethylcarboxylic acid derivatives. EP 1,666,447 A1

Nishii, Sadao. Preparation of 2-ethyl-2-(hydroxymethyl)hexanoic acid. Jpn. Kokai Tokkyo Koho (1989), JP 01139544

Die Verbindung der Formel 2a lässt sich beispielsweise herstellen indem man racemisches 2-butyl-2-ethyl-1,3-propandiol mit chiralen Basen, wie zum Beispiel Chinin, in einem geeigneten Lösungsmittel oder Lösungsmittelgemisch, wie zum Beispiel Toluol, n-Butylacetat oder Aceton/Wasser, umsetzt. Die Reaktionstemperatur der Racematspaltung beträgt dabei 0 °C bis 100°C, bevorzugt von 20 °C bis 60 °C. Die Reaktionszeit liegt im allgemeinen bei 2 bis 24 Stunden, je nach Zusammensetzung des Gemisches und des gewählten Temperaturbereiches. Die erzielten Enantiomerenüberschüsse (ee) liegen je nach gewählten Bedingungen zwischen 20 und 80% ee. Höhere ee Werte können erzielt werden, wenn die erhaltene Verbindung der Formel 2a anschließend in einem geeigneten Lösungsmittel oder Lösungsmittelgemisch wie zum Beispiel Toluol, n-Butylacetat/Heptan oder Aceton/Wasser umkristallisert wird. Die erzielten Enantiomerenüberschüsse (ee) liegen je nach gewählten Bedingungen zwischen 80 und 99% ee.

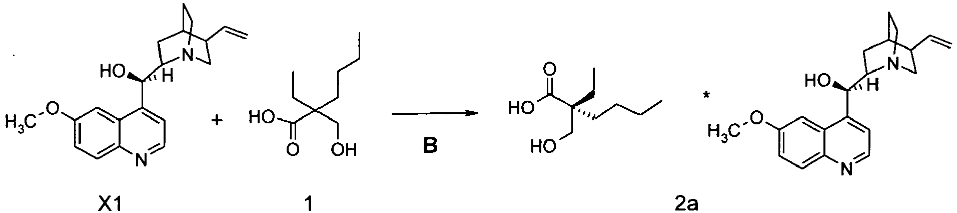

Die Verbindung der Formel 2 wird anschließend durch wässrige Aufarbeitung und Extraktion mit einem geeigneten Lösungsmittel, beispielsweise Toluol, Ethylacetat oder Dichlormethan aus der Verbindung der Formel 2a gewonnen.

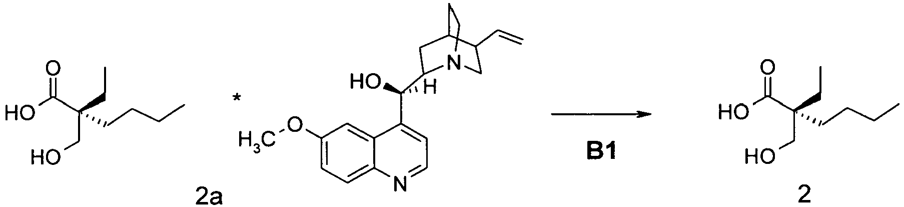

Alternativ kann die Verbindung 2 durch Racematspaltung mit der Verbindung der Formel X2 hergestellt werden. Die Verbindung der Formel 2 wird anschließend durch wässrige Aufarbeitung und Extraktion mit einem geeigneten Lösungsmittel, beispielsweise Toluol, Ethylacetat oder Dichlormethan aus der Verbindung der Formel 2b gewonnen.

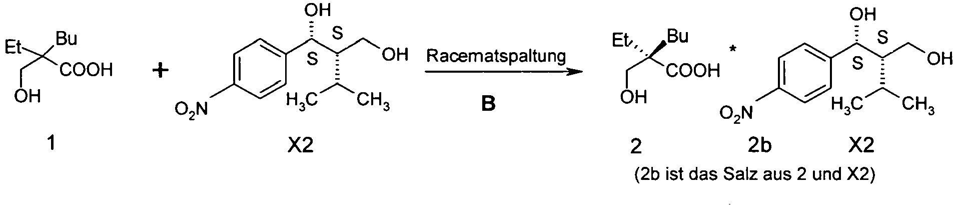

Das Enantiomer (Formel 2c) der Verbindung 2 kann durch Racematspaltung mit dem Antipoden (Formel X3) der Verbindung X2 hergestellt werden.

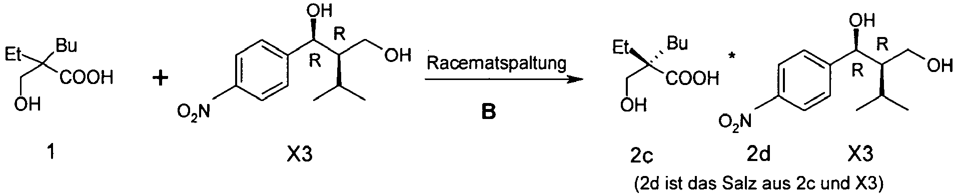

Die Verbindungen der Formel 3 und 3a lassen sich beispielsweise herstellen indem man die Verbindung der Formel 2 oder 2c z. B. mit Bromwasserstoffsäure ohne oder mit einem geeigneten Lösungsmittel oder Lösungsmittelgemisch, wie zum Beispiel Toluol, umsetzt. Die Reaktionstemperatur der beträgt dabei 40 °C bis 120°C, bevorzugt von 60 °C bis 100 °C. Die Reaktionszeit liegt im allgemeinen bei 2 bis 24 Stunden, je nach Zusammensetzung des Gemisches und des gewählten Temperaturbereiches. Die erzielten Ausbeuten liegen je nach gewählten Bedingungen zwischen 60 und 90%. 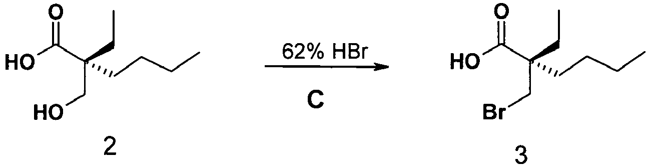 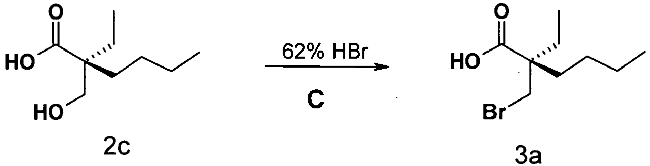 Die Herstellung des Racemates gelingt analog indem man die Verbindung der Formel 1 z. B. mit Bromwasserstoffsäure analog den oben genannten Bedingungen umsetzt.
Die erhaltenen Verbindungen der Formel 3 und 3a bzw. deren Gemisch werden anschließend durch wässrige Aufarbeitung und Extraktion mit einem geeigneten Lösungsmittel, beispielsweise Ethylacetat oder Dichlormethan aus dem Reaktionsgemisch abgetrennt. Eine Reinigung kann mit Hilfe einer Vakuumdestillation durchgeführt werden.
Weiterhin können die Verbindungen der Formel 3 und 3a bzw. deren Gemische nach den literaturbekannten Verfahren gewonnen werden.

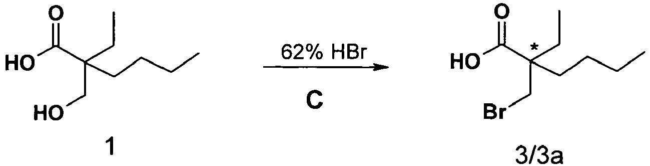

Mitsuda, Masaru; Oguro, Kazumi; Watabe, Kazuhiko; Hayano, Tetsuji. Preparation of 2-substituted-2-(hydroxymethyl)carboxylic acids (esters) and their intermediates. ; Jpn. Kokai Tokkyo Koho (2006), JP 2006219404

Crocq, Veronique; Roussel, Patrick. Process for preparation of new chiral compounds derived from esters of hexanoic acid, and their use in the synthesis of the chiral 2-(bromomethyl)-2-ethylhexanoic acid.; FR 2849024

Alternativ kann die Verbindung der Formel 3 oder 3a direkt aus den Salzen, wie z.B. 2a, 2b und 2d analog den beschriebenen Bedingungen gewonnen werden.

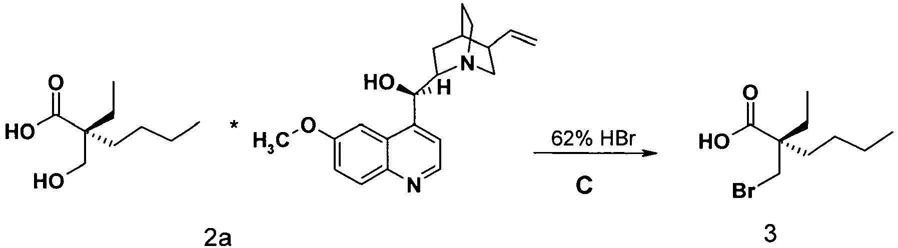

Die Verbindung der Formel 5 lässt sich beispielsweise herstellen indem man das Bromid der Verbindung der Formel 3 z. B. mit Kaliumthioacetat in einem geeigneten Lösungsmittel oder Lösungsmittelgemisch, wie zum Beispiel Toluol oder Aceton umsetzt. Die Reaktionstemperatur der beträgt dabei 0 °C bis 100°C, bevorzugt von 20 °C bis 40 °C. Die Reaktionszeit liegt im allgemeinen bei 2 bis 24 Stunden, je nach Zusammensetzung des Gemisches und des gewählten Temperaturbereiches. Die erhaltenen Verbindungen der Formel 4 werden anschließend durch wässrige Aufarbeitung und Extraktion mit einem geeigneten Lösungsmittel, beispielsweise Ethylacetat oder Dichlormethan aus dem Reaktionsgemisch abgetrennt. Bevorzugterweis wird das Reaktionsprodukt nicht isoliert, sondern direkt in einem geeigneten Lösungsmittel oder Lösungsmittlegemisch, wie z.B. THF/Toluol mit einem Reduktionsmittel wie z. B. Lithiumaluminiumhydrid (LAH) zur Verbindung der Formel 5 reduziert. Die Reaktionstemperatur beträgt dabei 0 °C bis 100°C, bevorzugt 0 °C bis 40 °C. Nach wässriger Aufarbeitung und Extraktion mit einem geeigneten Lösungsmittel, beispielsweise Ethylacetat, Toluol oder Dichlormethan wird das Produkt aus dem Reaktionsgemisch abgetrennt. Eine Reinigung kann mit Hilfe einer Vakuumdestillation durchgeführt werden.

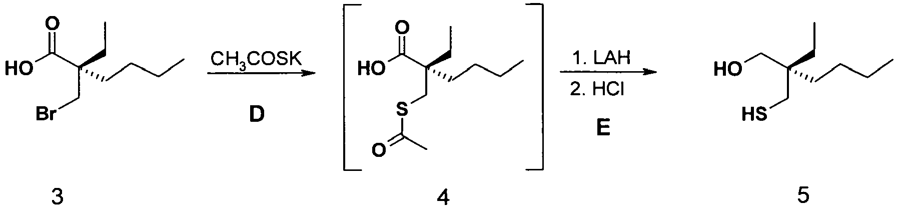

Die Darstellung der Verbindung der Formel 5a gelingt analog.

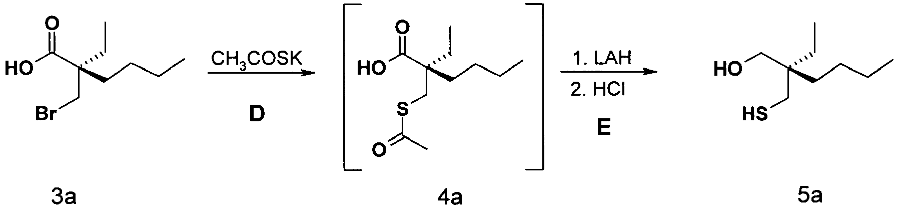

Eine Ausführungsform der Erfindung bezieht sich weiterhin auch auf einzelne Reaktionsschritte sowie Zwischenprodukte dieses Verfahrens zur Herstellung der Verbindung der Formel 10, das folgende Schritte aufweist:
Die Verbindung der Formel 10 lässt sich beispielsweise herstellen indem man die Verbindung der Formel 8 mit einem Thiol der Formel 35, in Gegenwart einer geeigneten, wässrigen Base, wie zum Beispiel Natriumcarbonat, Kaliumcarbonat, Caesiumcarbonat, umsetzt.
Die Reaktionstemperatur beträgt dabei von 40 °C bis 140 °C, bevorzugt von 60 °C bis 120°C. Die Reaktionszeit liegt im Allgemeinen bei 3 bis 24 Stunden, je nach Zusammensetzung des Gemisches und des gewählten Temperaturbereiches.

Die erhaltene Gemisch der Verbindungen der Formel 30/30a und 10/10a wird durch alkalische Hydrolyse der Verbindung der Formel 30/30a, z.B. mit Natriummethylat in Methanol oder methanolischer Kaliumhydroxid-Lösung, vollständig in die Verbindung der Formel 10/10a überführt und anschließend durch wässrige Aufarbeitung und Extraktion mit einem geeigneten Lösungsmittel, beispielsweise Toluol, Ethylacetat oder Dichlormethan aus dem Reaktionsgemisch abgetrennt.

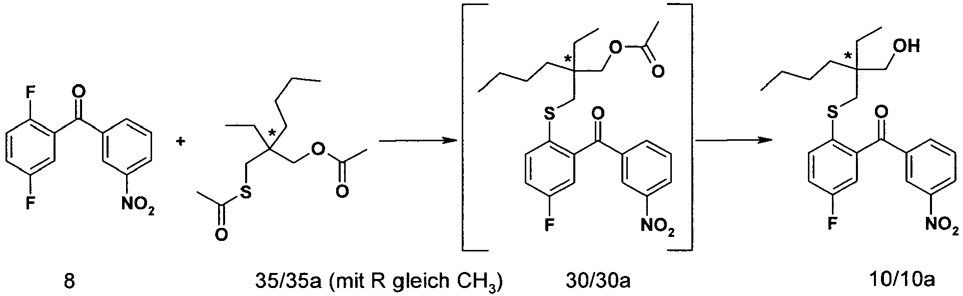

Der Stern an einem Kohlenstoffatom in der Verbindung der Formel 10 bedeutet, dass das jeweilige Kohlenstoffatom chiral ist und die Verbindung entweder als R- oder S-Enantiomer oder als Gemisch der beiden Enantiomeren vorliegt.

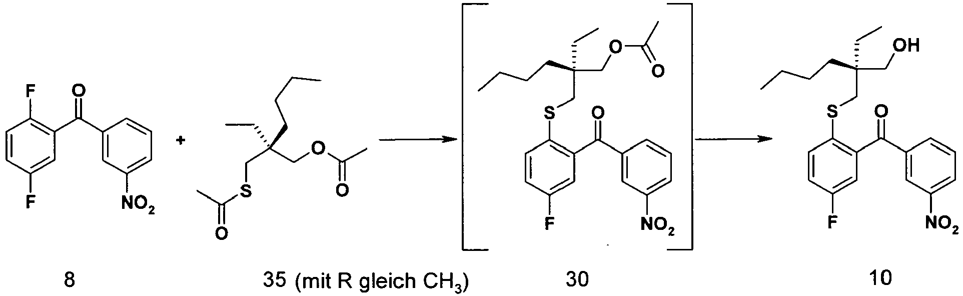

Bevorzugt wird die Verbindung 10 oder. 10a in enantiomerenreiner Form wie zum Beispiel durch Umsetzung der Verbindung der Formel 8 mit der Verbindung der Formel 35 unter den angegebenen Reaktionsbedingungen hergestellt. Die Herstellung der Verbindung der Formel 10a gelingt analog durch Umsetzung mit der Verbindung der Formel 5a.

Die Verbindung der Formel 35 lässt sich beispielsweise herstellen indem man das Bromid der Verbindung der Formel 3 z. B. mit Kaliumthioacetat in einem geeigneten Lösungsmittel oder Lösungsmittelgemisch, wie zum Beispiel Toluol oder Aceton umsetzt. Die Reaktionstemperatur der beträgt dabei 0 °C bis 100°C, bevorzugt von 20 °C bis 40 °C. Die Reaktionszeit liegt im allgemeinen bei 2 bis 24 Stunden, je nach Zusammensetzung des Gemisches und des gewählten Temperaturbereiches. Die erhaltenen Verbindungen der Formel 4 werden anschließend durch wässrige Aufarbeitung und Extraktion mit einem geeigneten Lösungsmittel, beispielsweise Ethylacetat oder Dichlormethan aus dem Reaktionsgemisch abgetrennt. Bevorzugterweis wird das Reaktionsprodukt nicht isoliert, sondern direkt in einem geeigneten Lösungsmittel oder Lösungsmittlegemisch, wie z.B. THF/Toluol mit einem Reduktionsmittel wie z. B. Lithiumaluminiumhydrid zur Verbindung der Formel 5 reduziert. Die Reaktionstemperatur beträgt dabei 0 °C bis 100°C, bevorzugt 0 °C bis 40 °C. Nach wässriger Aufarbeitung und Extraktion mit einem geeigneten Lösungsmittel, beispielsweise Ethylacetat, Toluol oder Dichlormethan wird das Produkt mit einem Säurechlorid oder säureanhydrid unter literaturbekannten Bedingungen umgesetzt. Bevorzugterweise wird das Reaktionsgemisch nach erfolgter Reduktion direkt mit einem Säurehalogenid oder Carbonsäureanhydrid, wie z. B. Acetylchlorid bzw. Acetanhydrid, hydrolysiert und anschließend wässrig aufgearbeitet. Eine Reinigung kann mit Hilfe einer Vakuumdestillation durchgeführt werden. 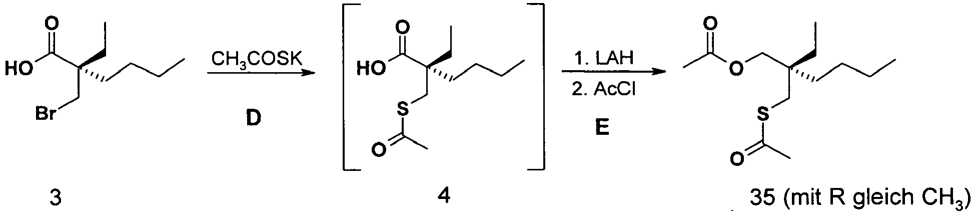 Die Herstellung der Verbindung der Formel 35a bzw. des Gemisches von 35/35a gelingt analog den für die Verbindung der Formel 35 genannten Bedingungen.

Eine Ausführungsform der Erfindung bezieht sich weiterhin auch auf einzelne Reaktionsschritte sowie Zwischenprodukte dieses Verfahrens zur Herstellung der Verbindungen der Formel 15, 15a, 15b und 15c, das folgende Schritte aufweist:
Die Verbindung der Formel 11/11a lässt sich beispielsweise herstellen indem man die Verbindung der Formel 10/10a mit einem geeigneten Reduktionsmittel, wie zum Beispiel Hydrophosphorigesäure/Iod, Natriumborhydrid/Aluminium-(III)-chlorid, Triethylsilan/Trifluoressigsäure, Isobutylaluminiumdichlorid, Butylsilan/Bortrifluorid, Polyhydroxymethylsilan (PHMS) oder Triethylsilan/Bortrifluorid ohne oder in einem geeigneten Lösungsmittel, wie z.B. Toluol, THF, Methyl-THF oder Dimethoxyethan umsetzt. Die Reaktionstemperatur beträgt dabei 20 °C bis 120 °C, bevorzugt von 40 °C bis 80 °C.
Die Reaktionszeit liegt im allgemeinen bei 2 bis 12 Stunden, je nach Zusammensetzung des Gemisches und des gewählten Temperaturbereiches. Die erhaltene Verbindunge der Formel 11/11a wird anschließend durch wässrige Aufarbeitung und Extraktion mit einem geeigneten Lösungsmittel, beispielsweise Toluol, Ethylacetat, Methyl-tert.-butylether oder Dichlormethan aus dem Reaktionsgemisch abgetrennt. 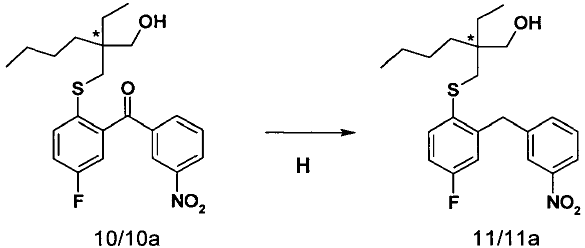 Der Stern an einem Kohlenstoffatom in den Verbindungen der Formel 10/10a und 11/11a bedeutet, dass das jeweilige Kohlenstoffatom chiral ist und die Verbindungen entweder als R-oder S-Enantiomer oder als Gemisch der beiden Enantiomeren vorliegt.
Die Herstellung der Verbindung der Formel 11 gelingt analog den für die Verbindungen der Formel 11/11a genannten Bedingungen. 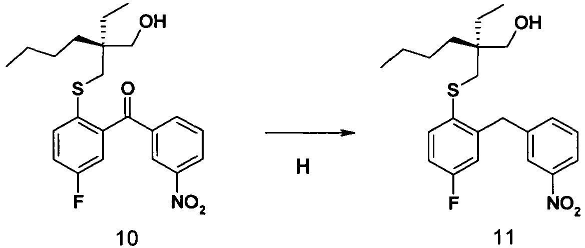 Die Verbindung der Formel 12 lässt sich beispielsweise herstellen indem man die Verbindung der Formel 11 mit einem geeigneten Oxydationsmittel, wie zum Beispiel Natriumperborat, Wasserstoffperoxid/Natriumwolframat, Wasserstoffperoxid/Molybdän-(IV)-oxiddichlorid, Oxone oder Wasserstoffperoxid/Acetonitril/Ethanol in einem geeigneten Lösungsmittel, wie z.B. Toluol, THF, Methyl-THF oder Dimethoxyethan umsetzt. Die Reaktionstemperatur beträgt dabei 0 °C bis 120 °C, bevorzugt von 20 °C bis 80 °C. 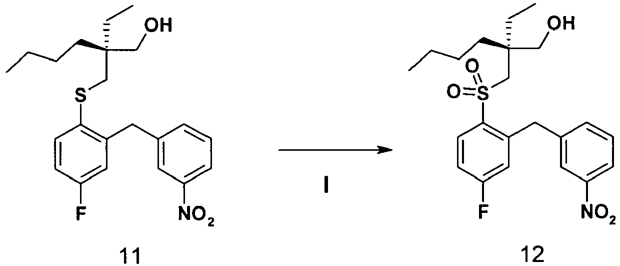 Die Reaktionszeit liegt im allgemeinen bei 2 bis 12 Stunden, je nach Zusammensetzung des Gemisches und des gewählten Temperaturbereiches. Die erhaltene Verbindunge der Formel 12 wird anschließend durch wässrige Aufarbeitung und Extraktion mit einem geeigneten Lösungsmittel, beispielsweise Toluol, Ethylacetat, Methyl-tert.-butylether oder Dichlormethan aus dem Reaktionsgemisch abgetrennt und kristallisiert.
Die Herstellung der Verbindung der Formel 12a gelingt analog den für die Verbindung der Formel 12 genannten Bedingungen. 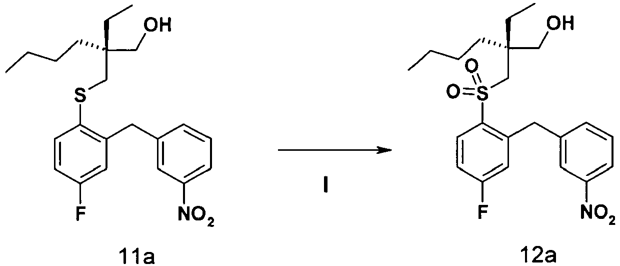 Die Verbindung der Formel 13 lässt sich beispielsweise herstellen indem man die Verbindung der Formel 12 mit einem geeigneten Oxydationsmittel, wie zum Beispiel Oxaylchlorid/DMSO, Schwefeltrioxid-Pyridin Komplex/DMSO, Pyridiniumdichromat, Periodan oder Natriumhypochlorid/TEMPO in einem geeigneten Lösungsmittel oder Lösungsmittelgemisch, wie z.B. Toluol, THF, Methyl-THF, Wasser oder Dimethoxyethan umsetzt. Die Reaktionstemperatur beträgt dabei 0 °C bis 100 °C, bevorzugt von 0 °C bis 40 °C.

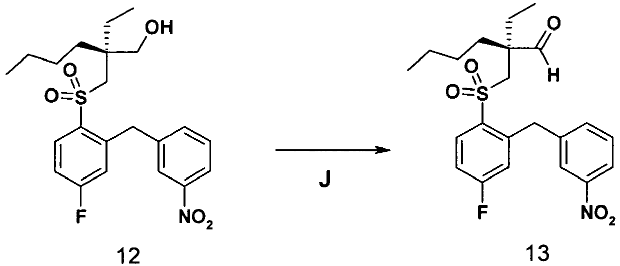

Die Reaktionszeit liegt im Allgemeinen bei 1 bis 4 Stunden, je nach Zusammensetzung des Gemisches und des gewählten Temperaturbereiches. Die erhaltene Verbindunge der Formel 13 wird anschließend durch wässrige Aufarbeitung und Extraktion mit einem geeigneten Lösungsmittel, beispielsweise Toluol, Ethylacetat, Methyl-tert.-butylether oder Dichlormethan aus dem Reaktionsgemisch abgetrennt und kristallisiert.
Die Herstellung der Verbindung der Formel 13a gelingt analog den für die Verbindung der Formel 13 genannten Bedingungen.

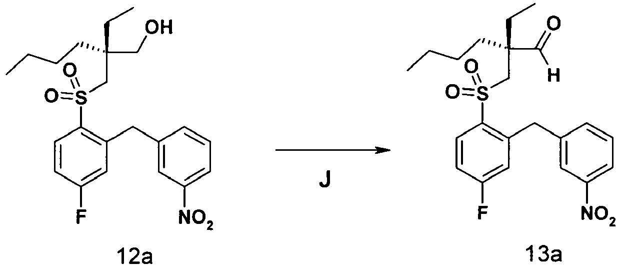

Die Verbindung der Formel 15 lässt sich beispielsweise herstellen indem man die Verbindung der Formel 13 mit einer geeigneten Base, wie zum Beispiel Kaliumcarbonat, Caesiumcarbonat, DBU, Natrium bzw. Kaliumethylat oder Natrium bzw. Kalium-tert.butylat einem geeigneten Lösungsmittel, wie z.B. 2-Propanol, Toluol, THF, Methyl-THF oder Dimethoxyethan umsetzt. Die Reaktionstemperatur beträgt dabei -70 °C bis 80 °C, bevorzugt von -20 °C bis 25 °C. Das entstandene Isomerengemisch kann anschließend über chromatographische Verfahren, wie z.B. Chromatographie auf Kieselgel und Toluol/Ethylacetat als mobile Phase, oder fraktionierte Kristallisation getrennt werden. 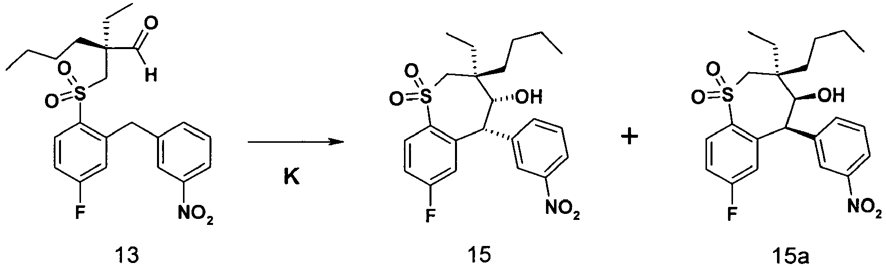 Die Herstellung der Verbindungen der Formel 15b und 15c gelingt analog den für die Verbindung der Formel 15 und 15a genannten Bedingungen.

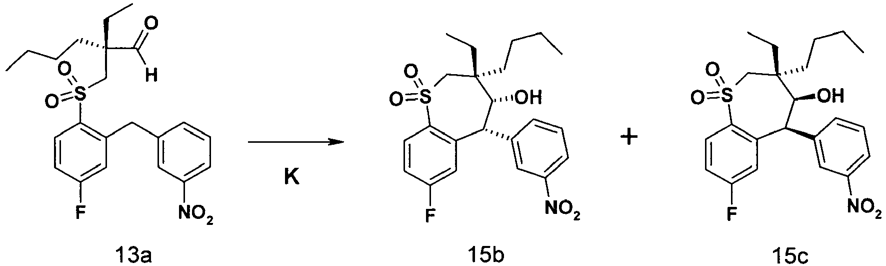

Eine Ausführungsform der Erfindung bezieht sich weiterhin auch auf einzelne Reaktionsschritte sowie Zwischenprodukte desVerfahrens zur Herstellung der Verbindungen der Formeln 17, 17a, 17b und 17c.

Die Verbindung der Formel 16 lässt sich beispielsweise herstellen indem man die Verbindung der Formel 15 mit einem geeigneten Reduktionsmittel, wie zum Beispiel Wasserstoff/Palladium auf Aktivkohle in einem geeigneten Lösungsmittel, wie z.B. Methanol, Ethanol, 2-Propanol, Dichlormethan, Toluol, THF, Methyl-THF oder Dimethoxyethan umsetzt. Die Reaktionstemperatur beträgt dabei 0 °C bis 80 °C, bevorzugt von 20 °C bis 50 °C.
Die Reaktionszeit liegt im Allgemeinen bei 2 bis 12 Stunden, je nach Zusammensetzung des Gemisches und des gewählten Temperaturbereiches. 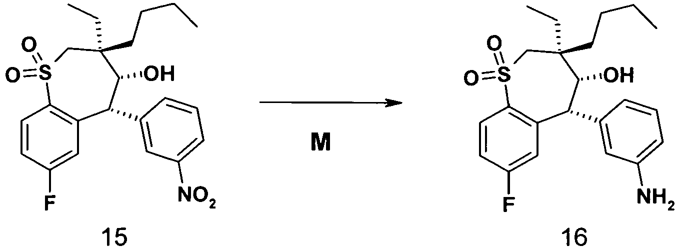 Die Herstellung der Verbindungen der Formel 16a, 16b und 16c bzw. deren Gemische gelingt analog den für die Verbindung der Formel 16 genannten Bedingungen. 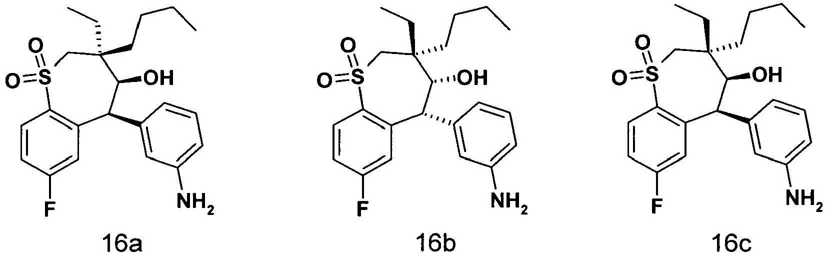 Die Verbindung der Formel 17 (nicht Teil der Erfindung) lässt sich beispielsweise herstellen indem man die Verbindung der Formel 16 mit Dimethylamin in einem geeigneten Lösungsmittel, wie z.B. Methanol, Ethanol, 2-Propanol, Toluol, THF, Methyl-THF oder Dimethoxyethan umsetzt. Die Reaktionstemperatur beträgt dabei 60°C bis 140 °C, bevorzugt von 80 °C bis 120 °C.
Die Reaktionszeit liegt im Allgemeinen bei 4 bis 24 Stunden, je nach Zusammensetzung des Gemisches und des gewählten Temperaturbereiches. Die erhaltene Verbindunge der Formel 17 wird anschließend mit einem geeigneten Lösungsmittel oder Lösungsmittelgemisch, beispielsweise Methanol, Ethanol, 2-Propanol, Methyl-tert.-butylether oder Diisopropylether aus dem Reaktionsgemisch kristallisiert.

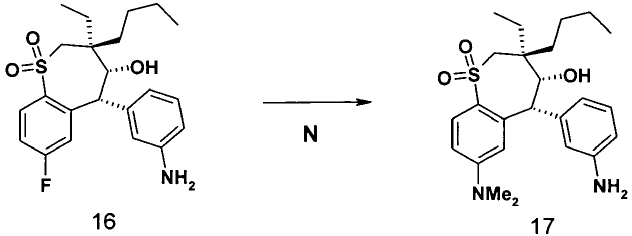

Die Herstellung der Verbindungen der Formel 17a, 17b und 17c bzw. deren Gemische gelingt analog den für die Verbindung der Formel 17 genannten Bedingungen. 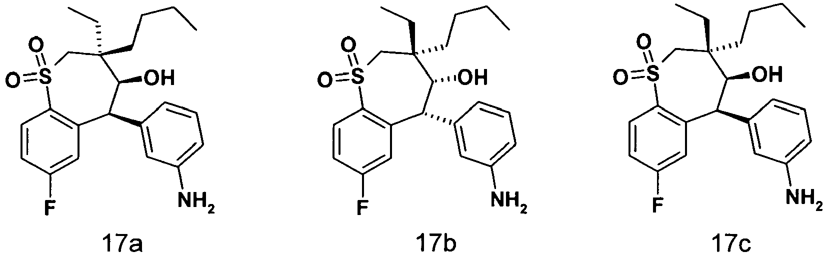 Alternativ kann das Verfahren bis zur Verbindung 17 bzw. 17a/17b und 17c wie folgt geführt werden:
Die Verbindung der Formel 31 lässt sich beispielsweise herstellen indem man die Verbindung der Formel 15 mit Dimethylamin in einem geeigneten Lösungsmittel, wie z.B. Methanol, Ethanol, 2-Propanol, Toluol, THF, Methyl-THF oder Dimethoxyethan umsetzt. Die Reaktionstemperatur beträgt dabei 60°C bis 140 °C, bevorzugt von 80 °C bis 120 °C.
Die Reaktionszeit liegt im Allgemeinen bei 4 bis 24 Stunden, je nach Zusammensetzung des Gemisches und des gewählten Temperaturbereiches. Die erhaltene Verbindunge der Formel 31 wird anschließend mit einem geeigneten Lösungsmittel oder Lösungsmittelgemisch, beispielsweise Methanol, Ethanol, 2-Propanol, Methyl-tert.-butylether oder Diisopropylether aus dem Reaktionsgemisch kristallisiert oder ohne Aufreinigung weiter umgesetzt.

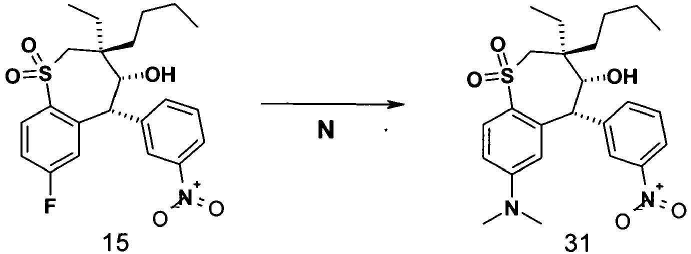

Die Herstellung der Verbindungen der Formel 31a, 31b und 31c bzw. deren Gemische gelingt analog den für die Verbindung der Formel 31 genannten Bedingungen.

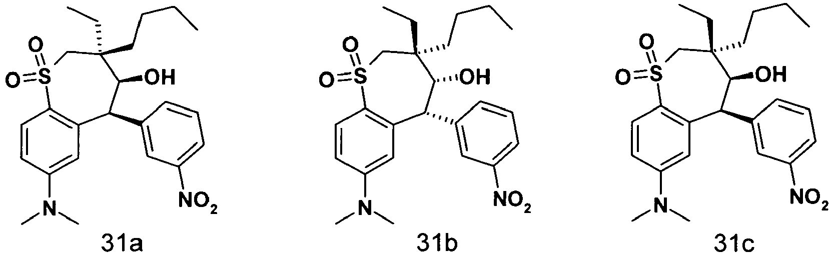

Die Verbindung der Formel 17 lässt sich beispielsweise herstellen indem man die Verbindung der Formel 31 mit einem geeigneten Reduktionsmittel, wie zum Wasserstoff/Palladium auf Aktivkohle in einem geeigneten Lösungsmittel, wie z.B. Methanol, Ethanol, 2-Propanol, Dichlormethan, Toluol, THF, Methyl-THF oder Dimethoxyethan umsetzt. Die Reaktionstemperatur beträgt dabei 0 °C bis 80 °C, bevorzugt von 20 °C bis 50 °C.
Die Reaktionszeit liegt im Allgemeinen bei 2 bis 12 Stunden, je nach Zusammensetzung des Gemisches und des gewählten Temperaturbereiches. Die erhaltene Verbindunge der Formel 17 wird anschließend mit einem geeigneten Lösungsmittel oder Lösungsmittelgemisch, beispielsweise Methanol, Ethanol, 2-Propanol, Methyl-tert.-butylether oder Diisopropylether aus dem Reaktionsgemisch kristallisiert.

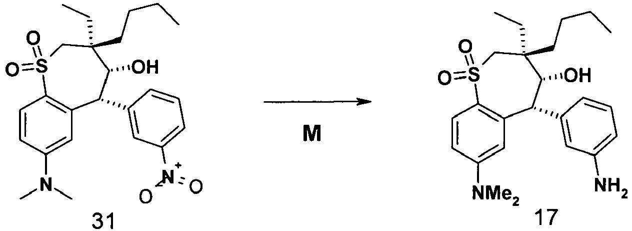

Verbindung 17 kann weiter wie folgt zur Herstellung der Verbindungen der Formel I genutzt werden.

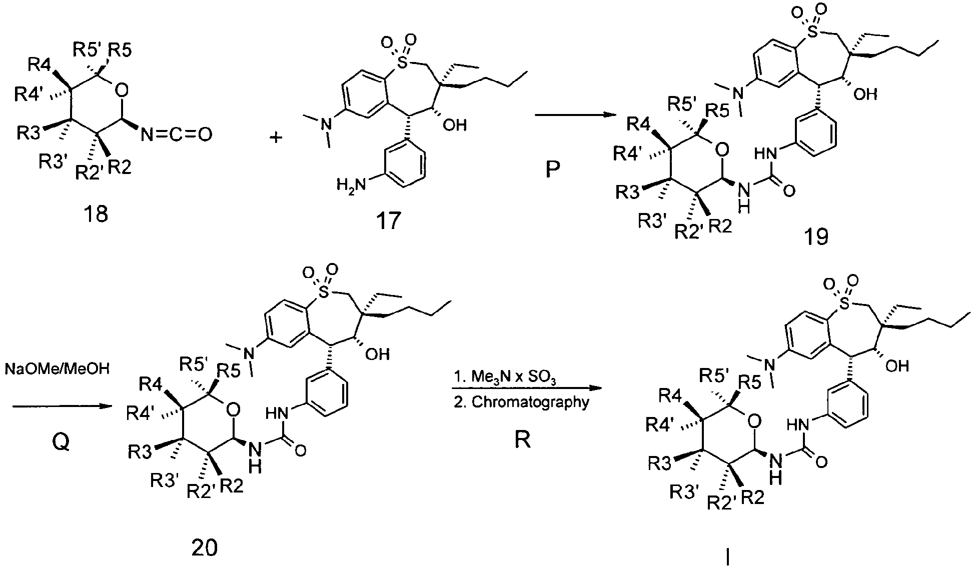

Verbindung 17a kann weiter wie folgt zur Herstellung der Verbindung der Formel Ia genutzt werden.

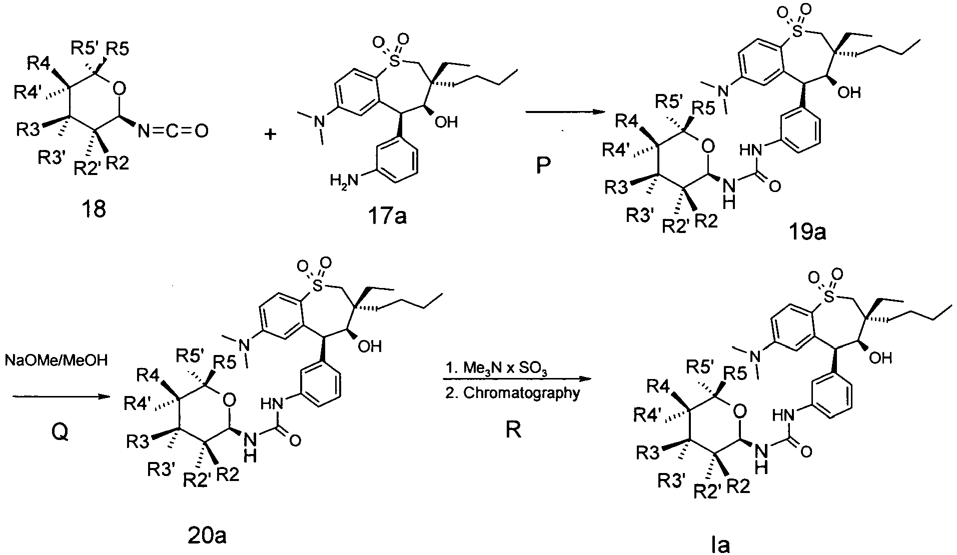

Verbindung 17b kann weiter wie folgt zur Herstellung der Verbindung der Formel Ib genutzt werden.

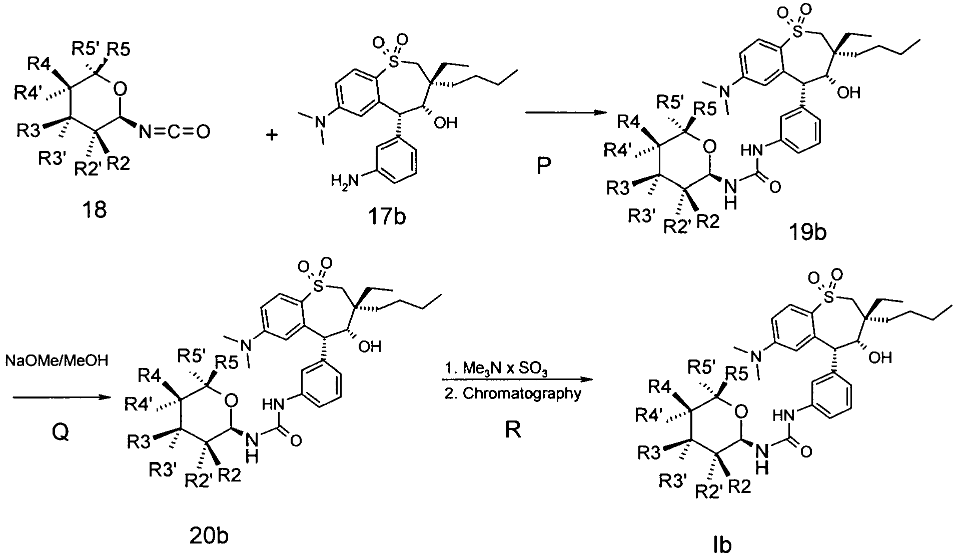

Verbindung 17c kann weiter wie folgt zur Herstellung der Verbindung der Formel Ic genutzt werden.

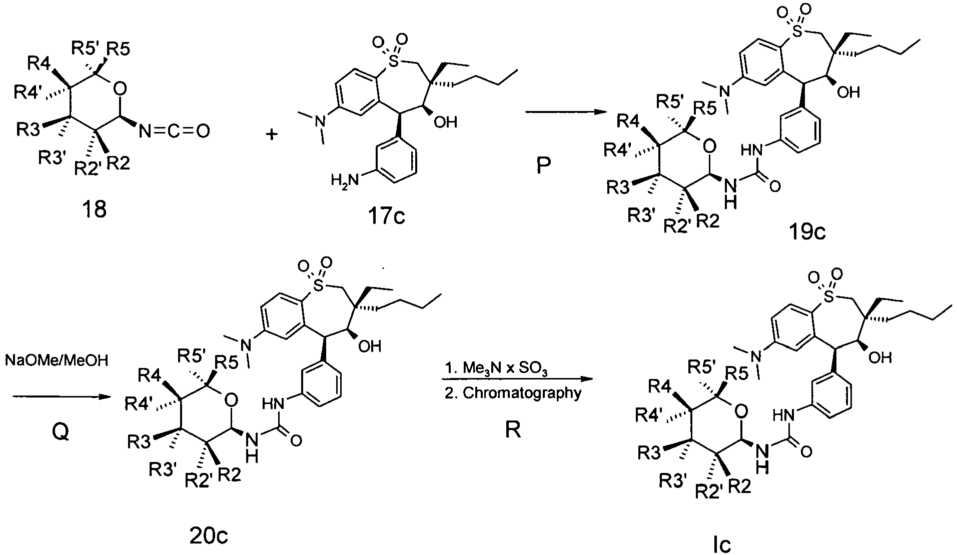

In einer bevorzugten Ausführungsform wird Verbindung 17 zur Herstellung der Verbindung 53 genutzt.

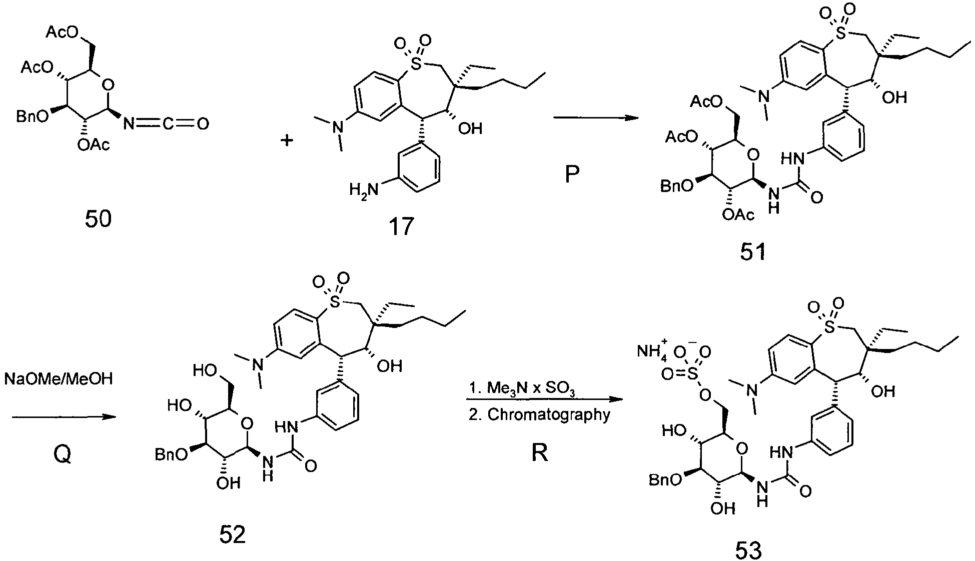

Die Herstellung der Verbindung 53a gelingt z. B. in analoger Form:

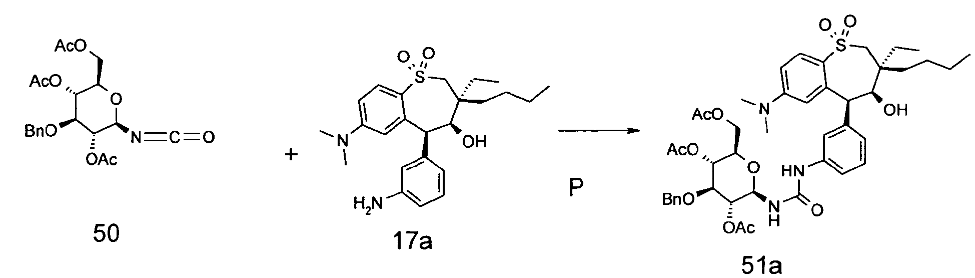

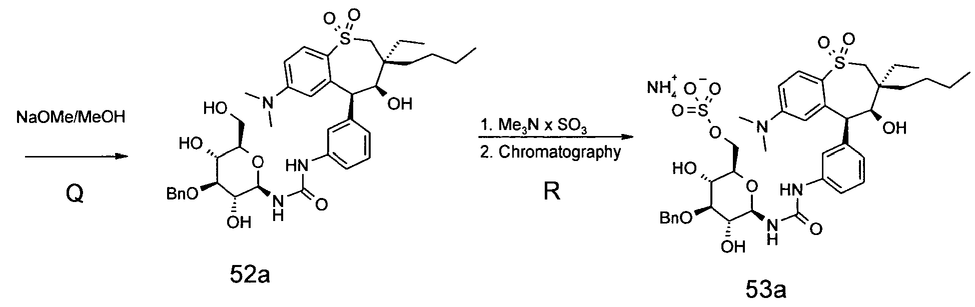

Die nachfolgend aufgeführten Beispiele dienen zur Erläuterung der Erfindung, ohne diese jedoch einzuschränken. die in der Tabelle angegebenen Verbindungen können nach dem obigen Verfahren hergestellt werden.

**Tabelle 1:**

| 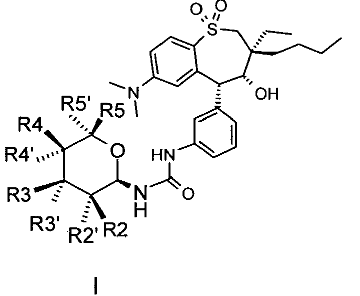 | | | | |
|---|---|---|---|---|
| **Bsp** | **R2, R2'** | **R3, R3'** | **R4, R4'** | **R5, R5'** |
| 1 | OH, H | OBn, H | OH, H | CH₂OH, H |
| 2 | OH, H | OBn, H | OH, H | CH₂OH, H |
| 3 | OH, H | OBn, H | OH, H | CH₂OBn, H |
| 4 | OH, H | OBn, H | OH, H | CH₂OSO₂OH, H |
| 5 | OH, H | OBn, H | OSO₂OH, H | CH₂OSO₂OH, H |
| 6 | OH, H | OBn, H | OH, H | CH₂OSO₂OH, H |

| | | | | |
|---|---|---|---|---|
| Et = Ethyl, Bu = n-Butyl, Bn = Benzyl | | | | |

Nachfolgend wird die Herstellung einiger Beispieles detailliert beschrieben, die übrigen Verbindungen der Formel I, Ia, Ib und Ic wurden analog erhalten:

### Experimenteller Teil:

### Beispiel 1 (Formel 52)

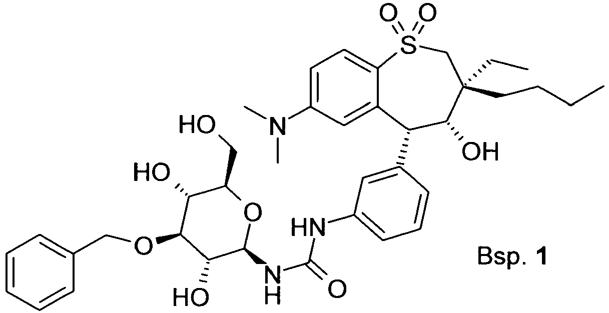

### Synthese von Beispiel 1:

### Verfahrensschritt A

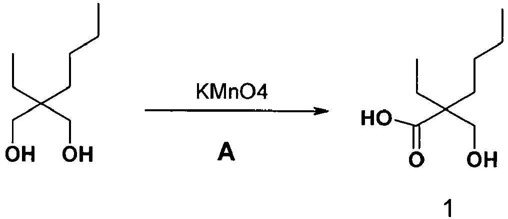

### Verfahrensschritt B

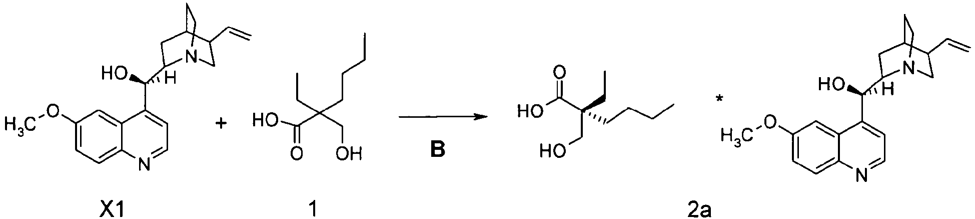

35.5g (204mmol) der Carbonsäure der Formel 1 und 63g (194mmol; 0.95äq.) Chinin werden in. 440ml n-Butylacetat und 220ml of n-Heptan suspendiert. Die Mischung wird auf 90°C erhitzt und für 15 Minuten bei dieser Temperatur gerührt. Anschließend wird auf 55°C, dann innerhalb von 12 Stunden auf Raumtemperatur abgekühlt und vom kristallisierten Chininsalz der Formel 1 2a abfiltriert.
Ausbeute: 62g (52%)
ee: 58% (RT: 6 Minuten; Chiralpak AD 250 x 4,6; n-Heptan/Ethanol 25:1; 30°C)

62g des Chininsalzes der Formel 2a werden in 400ml n-butylacetat und 400ml n-Heptan bei 100 bis 110°C gelöst und langsam über Nacht auf Raumtemperatur abgekühlt. Es wird vom ausgefallenen Feststoff der Formel 2a abgesaugt und im Vakuum getrocknet.
Ausbeute: 43g (70%)
ee: 94% (RT: 6 Minuten; Chiralpak AD 250 x 4,6; n-Heptan/Ethanol 25:1; 30°C)

### Verfahrensschritt C:

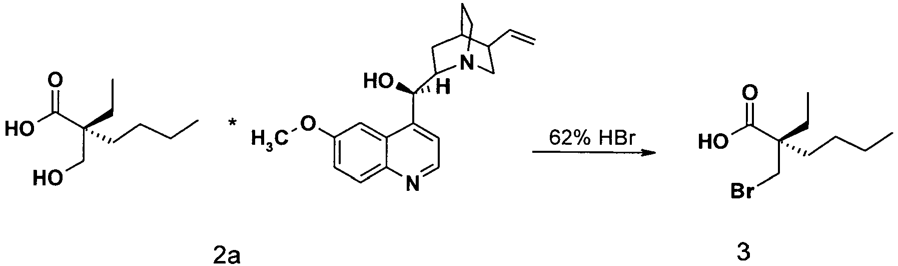

26g des Chininsalzes der Formel 2a werden in 110ml 62%iger HBr für 12 Stunden bei 100°C gerührt. Dannach ist die Umsetzung vollständig (DC Ethylacetat/n-Heptan 1:1). Die Lösung wird abgekühlt und mit 100ml Wasser und 100ml Toluol versetzt. Die wässrige Phase abgetrennt und die Toluolphase getrocknet und im Vakuum abdestilliert. Die Carbonsäure der Formel 3 wird über eine Kurzwegdestillation bei 2mbar und 140°C Manteltemperatur gereinigt.
Ausbeute: 11,1g (90%)
¹H-NMR (CDCl₃): 3,83 (s, 2H); 2,98 (s, 2H); 2,32 (s, 3H); 2,15 (s, 3H); 1,6 - 1,8 (m, 4H); 1,1 - 1,4 (m, 4H); 0,85 (t, 3H); 0,8 (t, 3H)

Alternativverfahrensschritt zu Verbindung 35a mit R gleich Methyl.

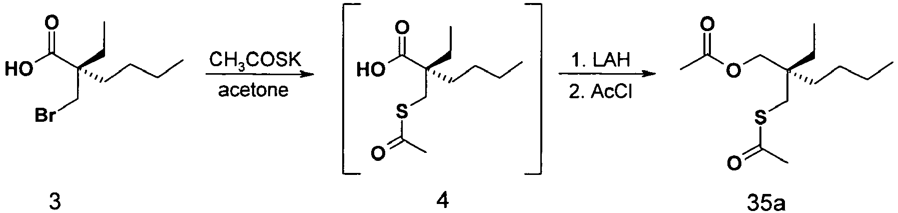

64g (0.56mol; 1.12eq.) Kaliumthioacetat werden in 400ml Aceton suspendiert.
118.57g (0.5mol) des Bromides der Formel 3, gelöst in 100ml Aceton warden zugegeben und die Lösung für 4 Stunden bei Raumtemperatur gerührt. Die Suspension wird mit 1500ml Toluol verdünnt und über 100g Silicagel filtriert. Das Filtrat wird bis auf ein Volumen von 1000ml im Vakuum eingeengt, auf 0°C abgekühlt und langsam mit 750ml (0.75mol) einer 1M LAH Lösung in THF versetzt. Die Lösung wird für 1-2 Stunden bei 0°C und über Nacht bei RT gerührt. Die Lösung wird auf 10°C abgekühlt und langsam mit 225ml Acetylchlorid versetzt. Es wird 1 Stunde nachgerührt und anschließend mit 250ml Toluol und 500ml Wasser versetzt. Die Phasen warden separiert und die wässrige Phase noch einmal mit 200ml Toluol extrahiert. Die vereingten Toluolphasen werden über Natriumsulfat getrocknet und das Lösungsmittel im Vakuum abdestilliert. Es wurde die Verbindung der Formel 35a (R gleich Methyl) erhalten.
Ausbeute: 123g (90%)
¹H-NMR (CDCl₃): 3,83 (s, 2H); 2,98 (s, 2H); 2,32 (s, 3H); 2,15 (s, 3H); 1,6 - 1,8 (m, 4H); 1,1 - 1,4 (m, 4H); 0,85 (t, 3H); 0,8 (t, 3H)

### Verfahrensschritte D und E:

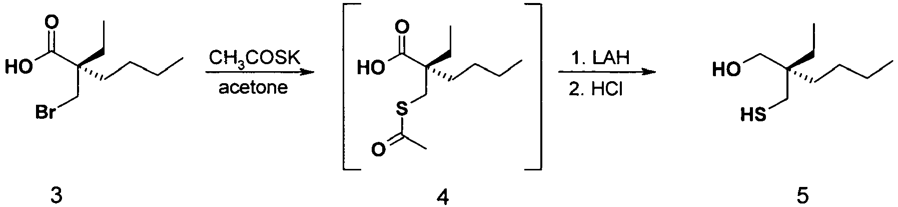

64g (0.56mol; 1.12eq.) Kaliumthioacetat werden in 400ml Aceton suspendiert.

118.57g (0.5mol) des Bromides der Formel 3, gelöst in 100ml Aceton wurden zugegeben und die Lösung für 4 Stunden bei Raumtemperatur gerührt. Die Suspension wird mit 1500ml Toluol verdünnt und über 100g Silicagel filtriert. Das Filtrat wird bis auf ein Volumen von 1000ml im Vakuum eingeengt, auf 0°C abgekühlt und langsam mit 750ml (0.75mol) einer 1M LAH Lösung in THF versetzt. Die Lösung wird für 1-2 Stunden bei 0°C und über Nacht bei RT gerührt. Die Lösung wird auf 10°C abgekühlt und langsam mit 700ml 2N Salzsäure versetzt. Es wird 1 Stunde nachgerührt und anschließend mit 250ml Toluol verdünnt. Die Phasen warden separiert und die wässrige Phase noch einmal mit 200ml Toluol extrahiert. Die vereingten Toluolphasen werden über Natriumsulfat getrocknet und das Lösungsmittel im Vakuum abdestilliert. Es wurde die Verbindung der Formel 5 erhalten.
Ausbeute: 103g (90%)
¹H-NMR (CDCl₃): 3,83 (s, 2H); 2,98 (s, 2H); 1,6 - 1,8 (m, 4H); 1,1 - 1,4 (m, 4H); 0,85 (t, 3H); 0,8 (t, 3H)

### Verfahrensschritt F:

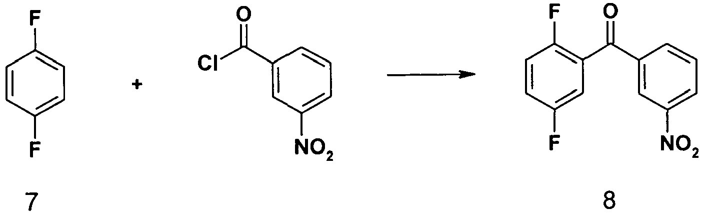

Zu einer Mischung aus 20g 3-Nitrobenzoylchlorid und 54ml 1,4 -Difluorbenzol werden innerhalb von 30 Minuten bei 20°C Innentemperatur 38,4g wasserfreies Aluminiumchlorid zugegeben, die Temperatur steigt dabei auf 30°C. Die Reaktionsmischung wird für 16 Stunden unter Rückfluß erhitzt. Dannach ist die Reaktion vollständig (DC Kontrolle mit Toluol/AcOEt/CH3CO2H 95:5:3).

Die Reaktionsmischung wird im auf 50°C abgekühlt und mit 40ml Essigsäureethylester versetzt. Die Suspension wird auf eine Mischung von 180ml Wasser und 30ml 2N-Salzsäure gegossen. Die Phasen werden getrennt und die wässrige Phase mit Essigsäureethylester nachextrahiert. Die vereingten organischen Phasen werden über Natriumsulfat getrocknet und das Lösungsmittel im Vakuum abgedampft. Das 2,4-Difluoro-3'-nitrobenzophenon der Formel 8 wird aus dem verbleibenden Rückstand mit 2-Propanol kristallisiert.
Ausbeute: 24,6g (86,6%)
1H-NMR (CDCl3): 8,63 (s, 1H); 8,49 (d, 1H); 8,15 (d, 1H); 7,71 (t, 1H); 7,15 - 7,45 (m, 3H)

### Alternativverfahrensschritt mit Verbindung 35a (R gleich Methyl)

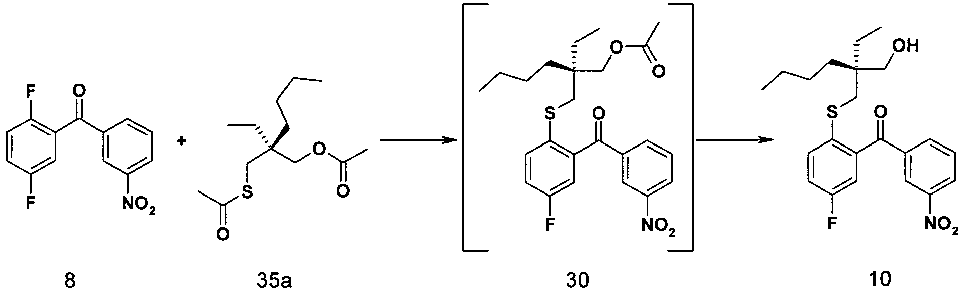

14,4g (1.15eq.) der Verbindung der Formel 8 und 1.9g Tetrabutylammoniumbromid werden in 80ml Toluol und 70ml einer 2M K₂CO₃ Lösung gelöst. Die Mischung wird unter Rückfluss erhitzt und innerhalb von 24 Stunden mit 14,5g der Verbindung der Formel 35a, gelöst in 30ml Toluol, versetzt. Die Reaktionsmischung wird noch für weitere 12 Stunden erhitzt. Anschließend wird auf RT abgekühlt, die Phasen getrennt und die organische Phase kurz andestilliert um Restmengen Wasser zu entfernen. Es werden 10ml Methanol und 2,5ml 30%iger Natriummethylat Lösung zugesetzt und 1,5 Stunden gerührt. Anschließend wird die Lösung aufkonzentriert und das Produkt chromatographisch (Eluent: Dichlormethan) gereingt. Es wurde die Verbindung der Formel 10 erhalten.
Ausbeute: 10,1g (57% bezogen auf Verbindung 35a)
1H-NMR (CDCl₃): 8,53 (s, 1H); 8,49 (d, 1H); 8,15 (d, 1H); 7,71 (t, 1H); 7,60 -7,68 (m, 1H); 3,45 (d, 2H); 2,83 (s, 2H); 1,05 -1,35 (m, 8H); 0,85 (t, 3H); 0,75 (t, 3H)

### Verfahrensschritt G:

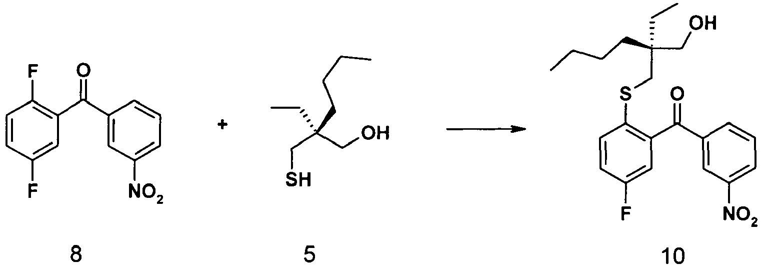

12,2g (1.15eq.) der Verbindung der Formel 8, 1.6g Tetrabutylammoniumbromid und 2g K₂CO₃ werden in 120ml Toluol 8 Stunden unter Rückfluss erhitzt. Anschließend wird auf RT abgekühlt, die Phasen getrennt, die Lösung aufkonzentriert und das Produkt chromatographisch (Eluent: Dichlormethan) gereingt. Es wurde die Verbindung der Formel 10 erhalten.
Ausbeute: 6,9 g (46% bezogen auf Verbindung 35a, hellgelbes Öl)
1H-NMR(CDCl₃): 8,53 (s, 1H); 8,49 (d, 1H); 8,15 (d, 1H); 7,71 (t, 1H); 7,60 -7,68 (m, 1H); 3,45 (d, 2H); 2,83 (s, 2H); 1,05 - 1,35 (m, 8H); 0,85 (t, 3H); 0,75 (t, 3H)

### Verfahrensschritt H:

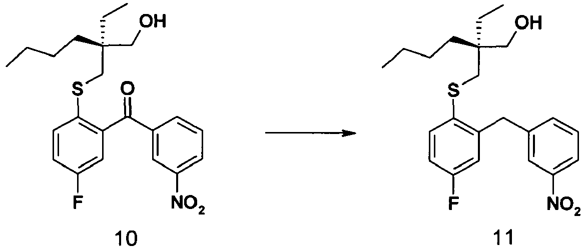

11g der Verbindung der Formel 11, 20g Triethylsilan und 25g Bortrifluorid-Diethylether Komplex werden für 8 Stunden bei 65°C Innentemperatur gerührt. Danach ist die Reduktion vollständig (DC: Toluol/Ethylacetat 10:1). Die Reaktionslösung wird auf RT abgekühlt und langsam mit 50ml einer 2M Natriumcarbonat-Lösung versetzt. Anschließend werden 100ml Ethylacetat zugegeben, die organische Phase im Vakuum eingeengt und das Produkt, dieVerbindung der Formel 11, chromatographisch (Eluent: Toluol/Ethylacetat 10:1) gereingt.
Ausbeute: 9,6 g (90,5%, hellgelbes Öl)
Rf = 0.4. C₂₂H₂₈FNO₃S (405,54). MS (M + H)+ = 406,54

### Verfahrensschritt I:

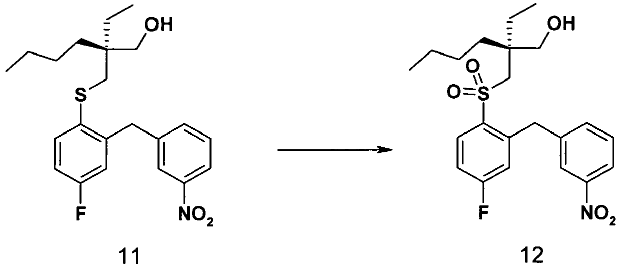

4g Kaliumcarbonat und 12g der Verbindung der Formel 11 werden in 80ml Ethanol, 20ml Acetonitril und 20ml Wasser gelöst. Die Lösung wird auf 5°C abgekühlt und mit 24ml 30%igem H₂O₂ innerhalb von 1 Stunde versetzt. Die Lösung wird über Nacht gerührt und zur Fällung des Rohproduktes mit 100ml Wasser versetzt. Das Rohprodukt wird abfiltriert, mit Wasser gewaschen und aus Diisopropylether kristallisiert. Die Verbindung der Formel 12 wurde erhalten.
Ausbeute: 11.65g (90%)
1H-NMR (CDCl₃): 8,05 - 8,15 (m, 3H); 7,55 -7,65 (m, 2H); 7,08 - 7,15 (m, 1H); 6,90 - 7,00 (m, 1H); 4,60 (s, 2H); 3,60 - 3,75 (m, 2H); 2,95 (s, 2H); 1,05 - 1,45 (m, 8H); 0,85 (t, 3H); 0,75 (t, 3H)

### Verfahrensschritt J:

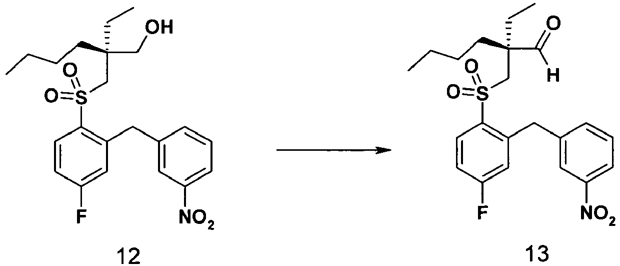

11.75g (27mol) der Verbindung der Formel 12 und 0.128g (0.022eq.) 4-Acetamido-TEMPO (4-Acetamido-2,2,6,6-tetramethylpiperidin-1-oxyl) werden in 160ml Dichloromethan gelöst. 1.5g of NaBr (0.54eq.) gelöst in 25ml Wasser und 4.45kg (2eq.) NaHCO₃, gelöst in 100ml Wasser, werden zugegeben. 20.1g (1.32eq.) einer 12.9%igen NaOCl werden kontinuierlich innerhalb von 2 Stunden zudosiert. Die Reaktionsmischung wird noch 15 Minuten nachgerührt und die vollständige Umsetzung über DC kontrolliert (Hepatan/Ethylacetat 2:1). Nach wässriger Aufarbeitung wird der Aldehyd der Formel 13 mit Diisopropylether kristallisiert.
Ausbeute: 10.5g (90%)
1 H-NMR (400MHz, CDCl3): 9,45 (s, 1H); 8,05 - 8,15 (m, 3H); 7,55 -7,65 (m, 2H); 7,08 - 7,15 (m, 1H); 6,90 - 7,00 (m, 1H); 4,60 (s, 2H); 3,20 (s, 2H); 1,55 - 2,05 (m, 4H); 1,05 - 1,35 (m, 4H);0,85 (t, 3H); 0,75 (t, 3H)

### Verfahrensschritt K und L:

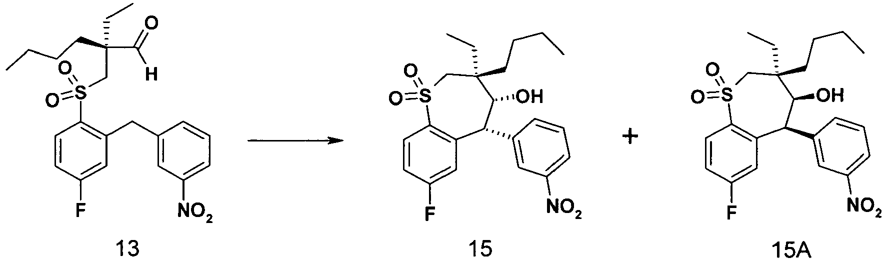

Eine Lösung von 9,3.g (21,4mmol) des Aldehydes der Formel 13 in 80ml THF wird bei 0°C mit 4,1ml (0.18eq.) einer 1M KOtBu in THF versetzt und für 1 Stunde bei dieser Temperatur nachgerührt. Die Reaktionslösung wird mit 0,25g Essigsäure (4,1mmol, 0.18eq.) neutralisiert und im Vakuum eingeengt. Die beiden Isomere (die Verbindungen der Formeln 15 und 15A) werden chromatographisch über Kieselgel getrennt (Eluent: Toluol/Ethylacetat 5:1).

Ausbeute Verbindung der Formel 15:4,1g (45%, hellgelber Feststoff)
Rf = 0.38. C₂₂H₂₆FNO₅S (435,52). MS (M + H)+ = 436,52
Ausbeute Verbindung der Formel 15A: 3,8g (41%, hellgelber Feststoff)
Rf= 0.49. C₂₂H₂₆FNO₅S (435,52). MS (M + H)+ = 436,52 Verbindungen der Formeln 17 und 17A (nicht Teil der Erfindung)

### Methode A:

### Verfahrensschritt M:

### Herstellung der Verbindung der Formel 16:

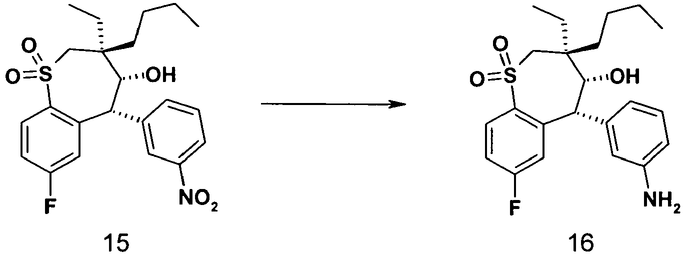

4g der Verbindung der Formel 15 werden in 40ml Dichlormethan/Ethanol :1 gelöst, mit 400mg Pd/C 5% versetzt und bis zum Ende der Wasserstoffaufnahme (3 - 4 Stunden) bei 3bar hydriert (DC Kontrolle: Ethylacetat/n-Heptan 2:1). Der Katalysator wird abfiltriert und die Lösungsmittel im Vakuum abdestilliert. Es wurde die Verbindung der Formel 16 erhalten.
Ausbeute: 3,7g (98%)
Rf = 0.48. C₂₂H₂₆FNO₃S (405,54). MS (M + H)+ = 406,54

### Verfahrensschritt N:

### Herstellung der Verbindung der Formel 17:

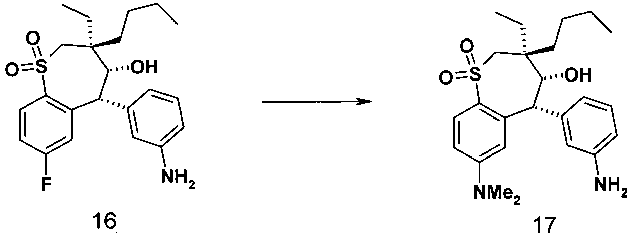

8g der Verbindung 16 werden in einem Druckbehälter vorgelegt und mit 50ml einer 33%igen Lösung von Dimethylamin in Ethanol versetzt. Der Druckbehälter wird Gasdicht verschlossen und die Lösung für mind. 8 Stunden auf 120°C erhitzt. Die Lösung wird abgekühlt und bis zur Kristallsiation langsam mit Wasser versetzt (10ml). Nach erfolgter Kristallisation werden zur vollständigen Fällung noch 50ml Wasser zugesetzt und die Suspension für 1 Stunde gerührt. Das Anilin (Verbindung 17) wird abfiltriert, gut mit Wasser gewaschen und im Vakuum getrocknet.
Ausbeute: 7,8g (91%, farbloser Feststoff)
¹H-NMR (400MHz, CDCl₃): 7,90 (d, 1H); 7,18 (t, 1H); 6,92 (d, 1H, b); 6,80 (s, 1H, b); 6,63 - 6,67 (m, 1H); 6,45 - 6,53 (m, 1H); 6,10 (s, 1H, b); 5,43 (s, 1H); 4,13 (s, 1H); 3,12 (d, 1H); 2,98 (d, 1H); 2,82 (s, 6H); 2,15 - 2,25 (m, 1H); 1,10 - 1,65 (m, 8H); 0,90 (t, 3H); 0,85 (t, 3H) C₂₄H₃₄N₂O₃S (437,54). MS (M + H)+ = 438,54

### Methode B:

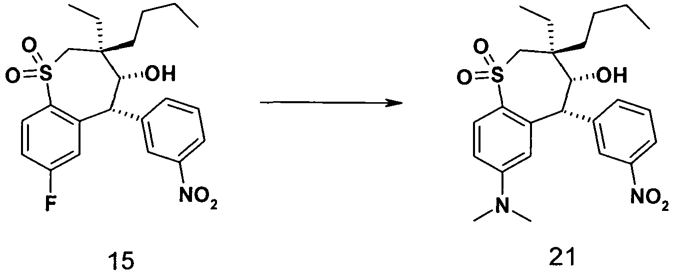

5g der Verbindung 15 werden in einem Druckbehälter vorgelegt und mit 50ml einer 33%igen Lösung von Dimethylamin in Ethanol versetzt. Der Druckbehälter wird Gasdicht verschlossen und die Lösung für mind. 8 Stunden auf 120°C erhitzt. Die Lösungsmittel werden abgedampft und der Rückstand über Kieselgel (Eluent: Ethylacetat/n-Heptan 2:1) chromatographiert.
Ausbeute: 4,76g (90%)
C₂₄H₃₂N₂O₅S (460,6). MS (M + H)+ = 461,6

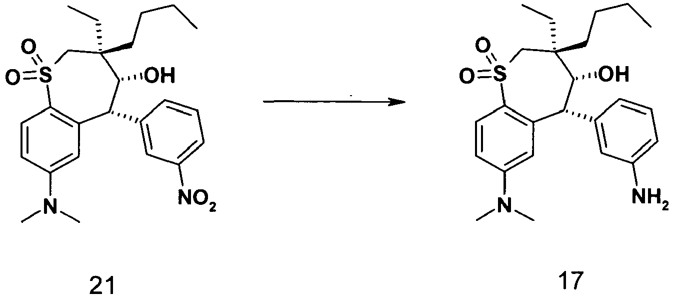

4g der Verbindung 21 werden in 40ml Ethanol gelöst, mit 400mg Pd/C 5% versetzt und bis zum Ende der Wasserstoffaufnahme (3 - 4 Stunden) bei 3bar hydriert (DC Kontrolle: Ethylacetat/n-Heptan 2:1). Der Katalysator wird abfiltriert und das Filtrat portionsweise, bis zur einsetztenden Kristallisation, mit Wasser versetzt. Es wird noch 30 Minuten nachgerührt und weitere 40ml Wasser nachgegeben. Der farblose Feststoff wird abfiltriert und im Vakuum getrocknet.
Ausbeute der Verbindung 17: 3,8g (91%, farbloser Feststoff)
¹H-NMR (400MHz, CDCl₃): 7,90 (d, 1H); 7,18 (t, 1H); 6,92 (d, 1H, b); 6,80 (s, 1H, b); 6,63 - 6,67 (m, 1H); 6,45 - 6,53 (m, 1H); 6,10 (s, 1H, b); 5,43 (s, 1H); 4,13 (s, 1H); 3,12 (d, 1H); 2,98 (d, 1H); 2,82 (s, 6H); 2,15 - 2,25 (m, 1H); 1,10 - 1,65 (m, 8H); 0,90 (t, 3H); 0,85 (t, 3H) C₂₄H₃₄N₂O₃S (437,54). MS (M + H)+ = 438,54

### Verfahrensschritt P:

### Herstellung der Verbindungen der Formeln 51 und 52:

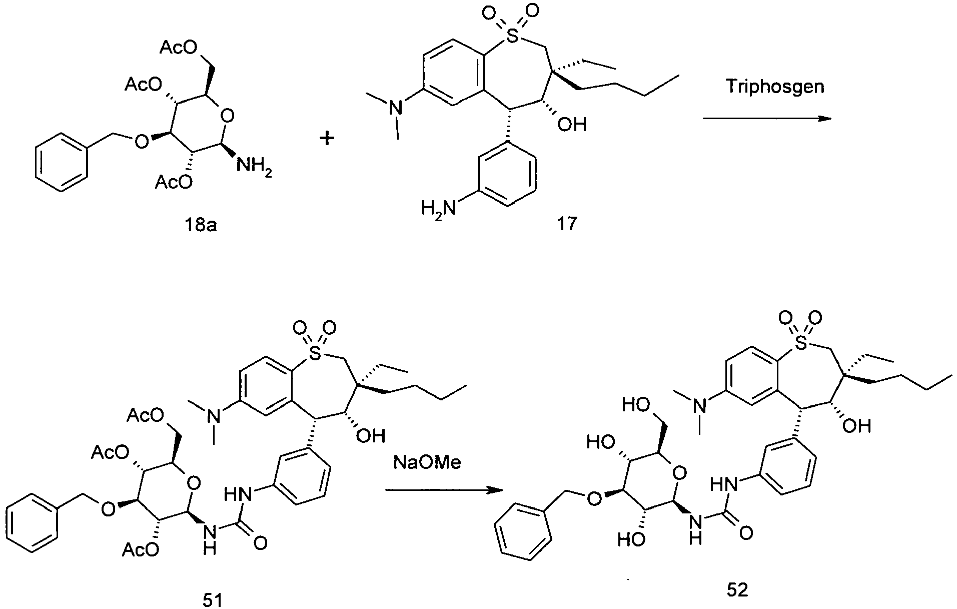

900 mg Triphosgen werden in 10 ml Methylenchlorid gelöst. Zu dieser Lösung tropft man innerhalb von 20 Minuten eine Lösung aus 3.0 g (7.6 mmol) Amin der Formel 18a und 3 ml N-Ethylmorpholin in 20 ml Methylenchlorid bei Raumtemperatur zu. Danach wird noch 1 Stunde gerührt und dann eine Lösung von 3.0 g (7.0 mmol) Anilin der Formel 17 (US 5,994,391), in 20 ml Methylenchlorid gelöst, langsam zugetropft.. Nach weiteren 30 Minuten ist die Reaktion beendet (DC-Kontrolle). Es wird zweimal mit ges. Natriumchloridlösung gewaschen, über Kieselgel filtriert und eingeengt und man erhält 7 g Rohprodukt der Formel 51. Dieses wird in 50 ml Methanol gelöst und mit 2 ml 1 M Natriummethanolat/Methanol Lösung versetzt. Nach 30 Minuten wird die Reaktionslösung mit 4 ml 0.5 M HCL/Methanol Lösung neutrallisiert und eingeengt. Der Rückstand wird mit Flashchromatographie gereinigt. Ausbeute 4.72 g (93 %) der Verbindung der Formel 52 als farbloser Feststoff. DC (Methylenchlorid/ Methanol/ konz. Ammoniak 30/5/1). R_{f}= 0.7. C₃₈H₅₁N₃O₉S (725.91). MS (M + H)⁺ = 726.38.

### Beispiel 2 und 3

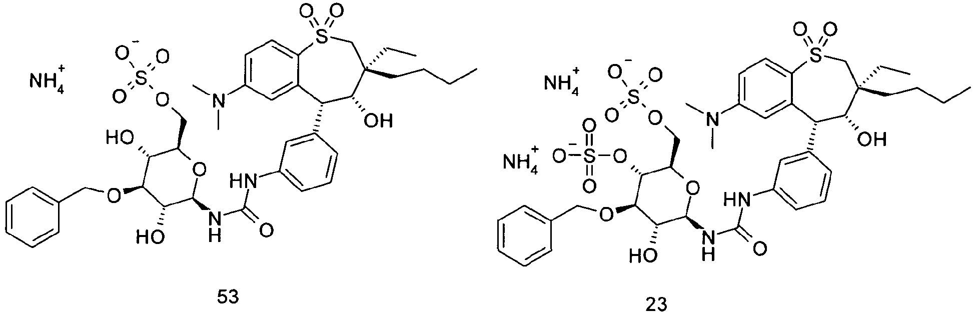

50.0 g (68.9 mmol) Beispiel 52 wird in 500 ml Pyridin gelöst und nach Zugabe von 17 g Pyridin-Sschwefeltrioxid-komplex 30 Minuten bei 60 ° C gerührt. Nach Zugabe von 400 ml Methanol wird am Rotationsverdampfer eingeengt. Der Rückstand wird noch einmal mit 300 ml Methanol abgeraucht und dann mit Flashchromatographie gereinigt. Ausbeute 38.4 g (68 %) der Verbindung der formel Ia als Ammoniumsalz. DC (Methylenchlorid/ Methanol/ konz. Ammoniak 30/5/1). R_{f}= 0.4. C₃₈H₅₁N₃O₁₂S₂ x NH₃ (823.00). MS (M + H)⁺ = 804.21. Als Nebenprodukt erhält man 4.0 g (7 %) des Disulfats der Formel 23 als doppeltes Ammoniumsalz. DC (Methylenchlorid/ Methanol/ konz. Ammoniak 30/5/1). R_{f}= 0.1. C₃₈H₅₀N₃O₁₅S₃ x 2NH₃ (920.09). MS (M + H)⁺ = 886.45.

Dieses Disulfat kann auch als Hauptprodukt erhalten werden, wenn man die doppelte Menge an Schwefeltrioxid-Komplex verwendet.

### Beispiel 4 (Formel 52a)

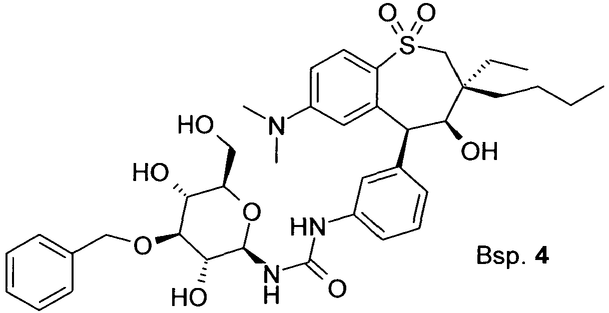

### Verfahrensschritt M:

### Herstellung der Verbindung der Formel 16A:

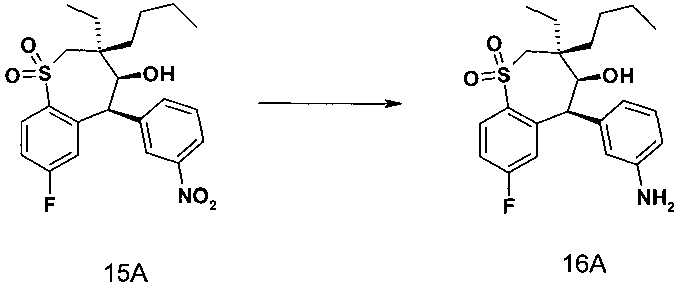

Die Verbindung 16A wurde analog Verbindung 16 hergestellt.
Ausbeute: 3,7g (98%)
¹H-NMR (400MHz, DMSO): 7,95 (m, 1H); 7,25 (t, 1H); 7,10 (t, 1H); 6,72 (d, 1H, b); 6,50 - 6,58 (m, 3H); 5,22 (d, 1H); 5,05 - 5,10 (m, 3H); 3,98 (d, 1H); 3,18 (d, 1H); 3,08 (d, 1H); 2,08 - 2,15 (m, 1H); 1,60 - 1,65 (m, 1H); 1.,05- 1,40 (m, 6H); 0,84 (t, 3H); 0,82 (t, 3H)

### Verfahrensschritt N:

### Herstellung der Verbindung der Formel 17A:

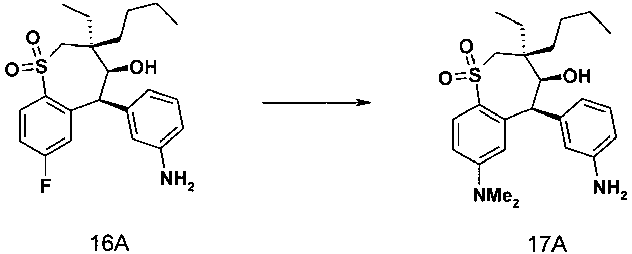

Die Verbindung 17A wurde analog Verbindung 17 hergestellt.
Ausbeute: 7,6g (88%, farbloser Feststoff)
¹H-NMR (400MHz, DMSO): 7,62 (d, 1H); 7,18 (t, 1H); 6,73 (d, 1H, b); 6,50 - 6,58 (m, 2H); 6,48 (d, 1H, b); 6,10 (s, 1H, b); 5,00 - 5,05 (m, 3H); 4,85 (d, 1H); 3,92 (d, 1H); 3,40 -3,50 (m, 1H); 3,00 (d, 1H); 3,03 (d,1H); 2,75 (s, 6H); 2,05 - 2,15 (m, 1H); 1,60 - 1,68 (m, 1H);1,32 - 1,40 (m, 1H); 1,00 - 1,25 (m, 6H);0,85 (t, 3H); 0,80 (t, 3H)

### Verfahrensschritt P:

### Herstellung der Verbindung der Formel 52a:

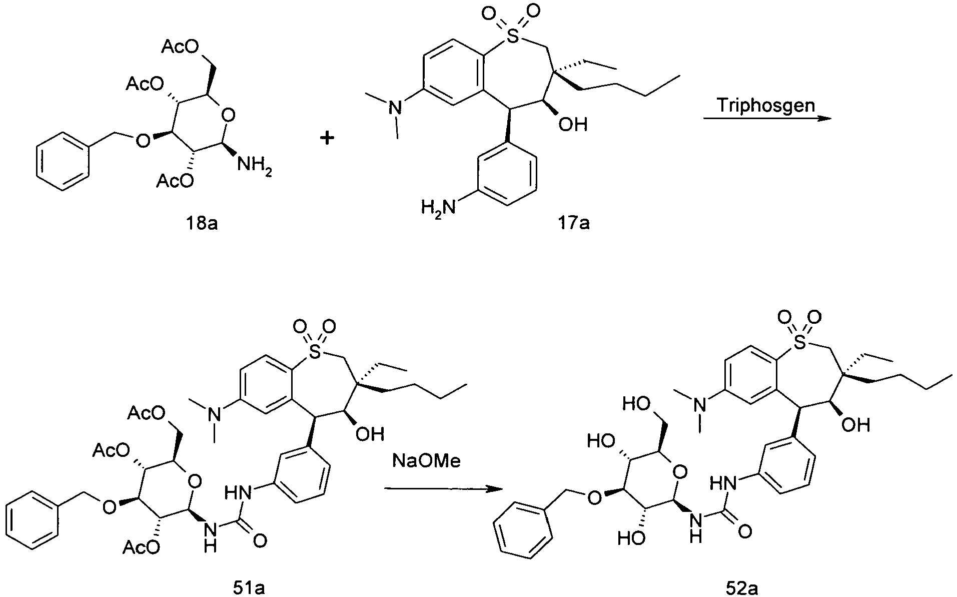

2.7 g Triphosgen werden in 30 ml Methylenchlorid gelöst. Zu dieser Lösung tropft man innerhalb von 20 Minuten eine Lösung aus 9.0 g (22.8 mmol) Amin der Formel 18a und 9 ml N-Ethylmorpholin in 60 ml Methylenchlorid bei Raumtemperatur zu. Danach wird noch 1 Stunde gerührt und dann eine Lösung von 9.0 g (21.0 mmol) Anilin der Formel 17a, in 50 ml Methylenchlorid gelöst, langsam zugetropft.. Nach weiteren 30 Minuten ist die Reaktion beendet (DC-Kontrolle). Es wird zweimal mit ges. Natriumchloridlösung gewaschen, über Kieselgel filtriert und eingeengt und man erhält 21 g Rohprodukt der Formel 51 a. Dieses wird in 100 ml Methanol gelöst und mit 5 ml 1 M Natriummethanolat/Methanol Lösung versetzt. Nach 30 Minuten wird die Reaktionslösung mit 10 ml 0.5 M HCL/Methanol Lösung neutrallisiert und eingeengt. Der Rückstand wird mit Flashchromatographie gereinigt. Ausbeute 14 g (92 %) der Verbindung der Formel 52a als farbloser Feststoff. DC (Methylenchlorid/ Methanol/ konz. Ammoniak 30/5/1). R_{f}= 0.65. C₃₈H₅₁N₃O₉S (725.91). MS (M + H)⁺= 726.38.

### Beispiel 5 und 6

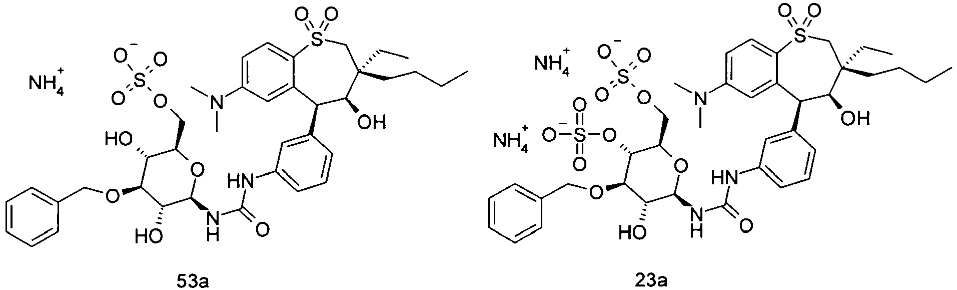

10.0 g (13.8 mmol) Beispiel 52a wird in 100 ml Pyridin gelöst und nach Zugabe von 3.5 g Pyridin-Sschwefeltrioxid-komplex 30 Minuten bei 60 ° C gerührt. Nach Zugabe von 100 ml Methanol wird am Rotationsverdampfer eingeengt. Der Rückstand wird noch einmal mit 100 ml Methanol abgeraucht und dann mit Flashchromatographie gereinigt. Ausbeute 7 g (64 %) der Verbindung der Formel 53a als Ammoniumsalz. DC (Methylenchlorid/ Methanol/ konz. Ammoniak 30/5/1). R_{f}= 0.35. C₃₈H₅₁N₃O₁₂S₂ x NH₃ (823.00). MS (M + H)⁺= 804.21.

Als Nebenprodukt erhält man 0.8 g (7 %) des Disulfats der Formel 23a als doppeltes Ammoniumsalz. DC (Methylenchlorid/ Methanol/ konz. Ammoniak 30/5/1). R_{f}= 0.1.
C₃₈H₅₁N₃O₁₅S₃ x 2NH₃ (920.09). MS (M + H)⁺ = 886.45.

Dieses Disulfat kann auch als Hauptprodukt erhalten werden, wenn man die doppelte Menge an Schwefeltrioxid-Komplex verwendet.

## Patentansprüche

1. Verfahren zur Herstellung der Verbindung der Formel I 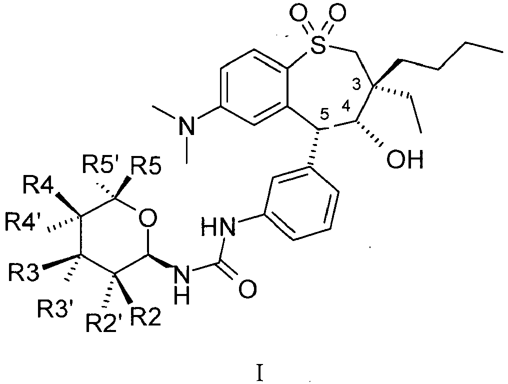 worin bedeuten
R2, R2', R3, R3', R4, R4', R5, R5' unabhängig voneinander H, Cl, Br, I, OH, -(CH₂)-OH, CF₃, NO₂ N₃, CN, S(O)ₚ-R6, O-S(O)ₚ-R6, (C₁-C₆)-Alkylen-S(O)ₚ-R6, (C₁-C₆)-Alkylen-O-S(O)ₚ-R6, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
Phenyl, -(CH₂)-Phenyl, -(CH₂)ₙ-Phenyl, O-Phenyl, O-(CH₂)ₘ-Phenyl, -(CH₂)-O-(CH₂)ₘ-Phenyl, wobei der Phenylring ein bis 3-fach substituiert sein kann mit F, Cl, Br, I, OH, CF₃, NO₂ CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
wobei immer mindestens einer der Reste R2, R2', R3, R3', R4, R4', R5, R5' die Bedeutung -O-(CH₂)ₘ-Phenyl oder -(CH₂)-O-(CH₂)ₘ-Phenyl besitzt, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R6 H, OH, (C₁-C₆)-Alkyl, NH₂, NH(C₁-C₆)-Alkyl, N((C₁-C₆)-Alkyl)₂;
n 2, 3, 4, 5, 6;
m 1,2,3,4,5,6;
p 0, 1, 2;
**dadurch gekennzeichnet, dass**
G) die Verbindung der Formel 5 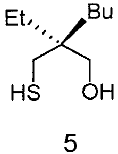 mit einer Verbindung der Formel 8 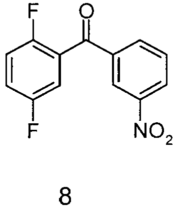 zur Verbindung der Formel 10 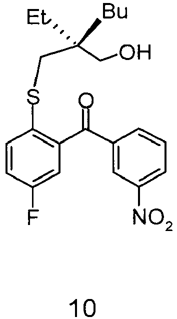 umgesetzt wird und dann in einem weiteren Verfahrensschritt
H) die Verbindung der Formel 10 in Gegenwart von BF₃ und Et₃SiH zu einer Verbindung der Formel 11 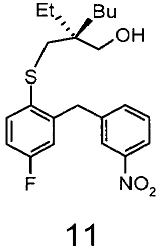 umgesetzt wird und dann in einem weiteren Verfahrensschritt
I) die Verbindung der Formel 11 in Gegenwart von H₂O₂ zu Verbindung 12 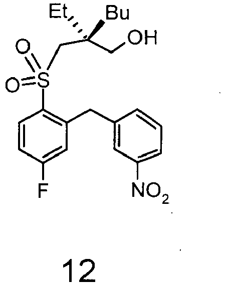 umgesetzt wird und dann in einem weiteren Verfahrensschritt
J) die Verbindung der Formel 12 in Gegenwart von TEMPO (2,2,6,6-Tetramethylpiperidinyloxyl) zu Verbindung 13 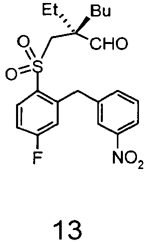 umgesetzt wird und dann in einem weiteren Verfahrensschritt
K) die Verbindung der Formel 13 in Gegenwart von tBuOK in THF zu Verbindung 15/15A 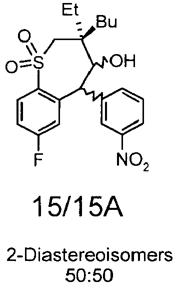 umgesetzt wird und dann in einem weiteren Verfahrensschritt
L) die Verbindung der Formel 15 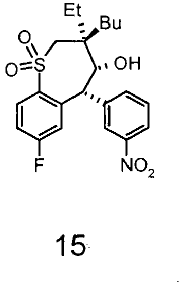 isoliert wird und dann in einem weiteren Verfahrensschritt
M) die Verbindung der Formel 15 mit Hilfe von H₂/Pd-C zu einer Verbindung der Formel 16 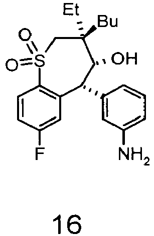 umgesetzt wird und dann in einem weiteren Verfahrensschritt
N) die Verbindung der Formel 16 in Gegenwart von HNMe₂ zu einer Verbindung der Formel 17/17A 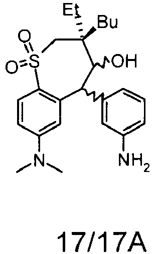 umgesetzt wird und dann in einem weiteren Verfahrensschritt
O) die Verbindung der Formel 17 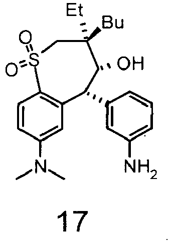 isoliert wird und dann in einem weiteren Verfahrensschritt
P) die Verbindung 17 mit der Verbindung 18, worin die Reste die oben angegebenen Bedeutungen haben 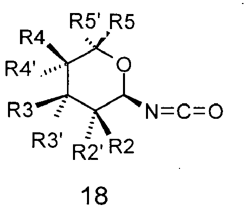 zu einer Verbindung der Formel 19 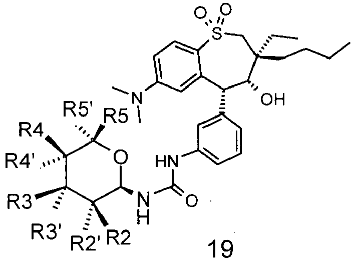 umgesetzt wird und dann optional in einem weiteren Verfahrensschritt
Q) die Verbindung der Formel 19 , worin die Reste die oben angegebenen Bedeutungen haben, zu einer Verbindung der Formel 20, worin die Reste die oben angegebenen Bedeutungen haben, 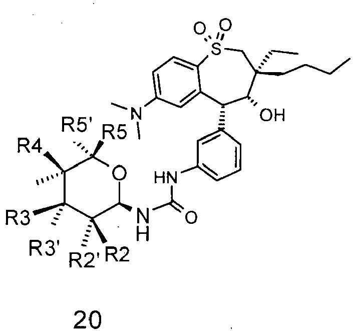 umgesetzt wird, wobei eventuell vorhandene Schutzgruppen abgespalten werden, und dann optional in einem weiteren Verfahrensschritt
R) die Verbindung der Formel 20, worin die Reste die oben angegebenen Bedeutungen haben, zu einer Verbindung der Formel Ia, worin die Reste die oben angegebenen Bedeutungen haben umgesetzt werden, 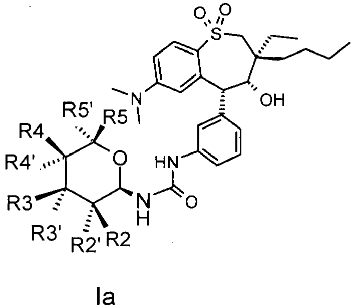 wodurch die Substituenten R2, R2', R3, R3', R4, R4', R5, R5' aus Formel 20 ausgetauscht werden können.

2. Verbindung der Formel 2a

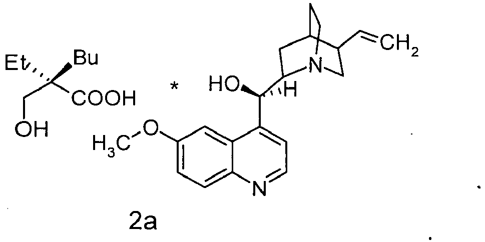

3. Verbindung der Formel 4

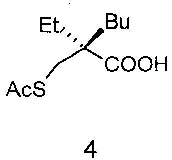

4. Verbindung der Formel 4a

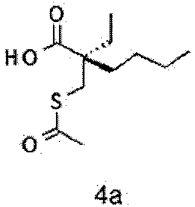

5. Verbindung der Formel 5

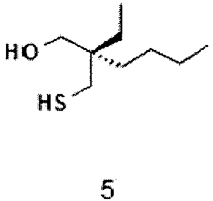

6. Verbindung der Formel 5a

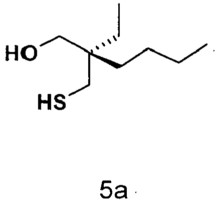

7. Racemat der Verbindungen der Formel 35/35a 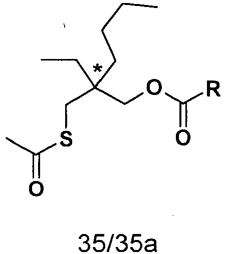 worin bedeuten
R (C₁-C₆)-alkyl.

8. Verbindung der Formel 35 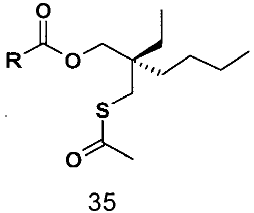 worin bedeuten
R (C₁-C₆)-alkyl.

9. Verbindung der Formel 35a 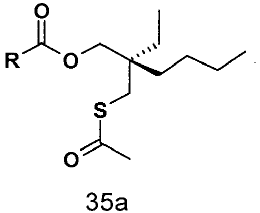 worin bedeuten
R (C₁-C₆)-alkyl.

10. Verbindung der Formel 8

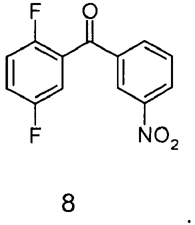

11. Verbindung der Formel 10

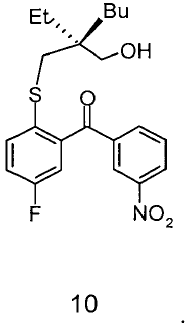

12. Verbindung der Formel 10a

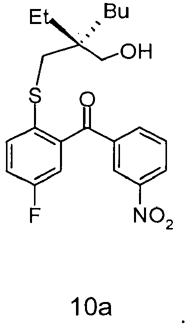

13. Verbindung der Formel 11

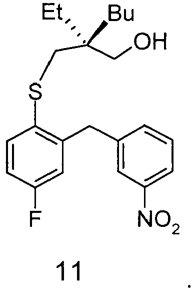

14. Verbindung der Formel 11a

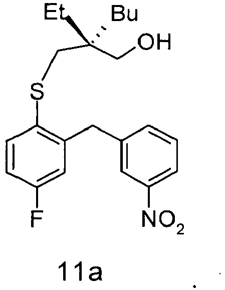

15. Verbindung der Formel 12

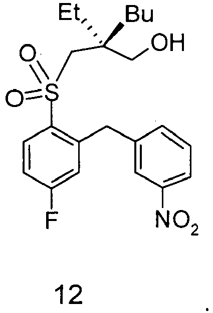

16. Verbindung der Formel 12a

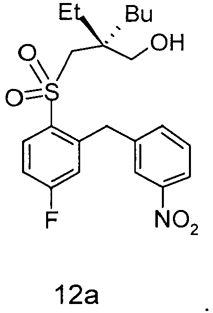

17. Verbindung der Formel 13

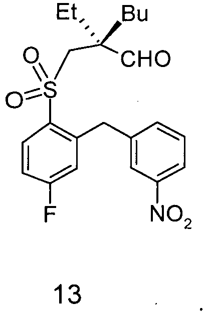

18. Verbindung der Formel 13a

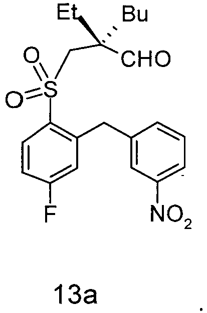

19. Verbindung der Formel 15

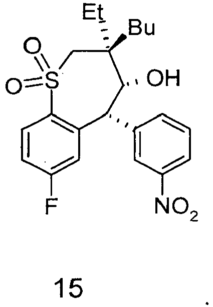

20. Verbindung der Formel 15B

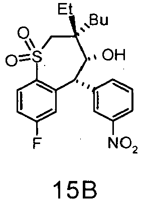

21. Verbindung der Formel 15C

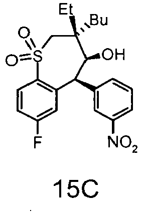

22. Verbindung der Formel 16

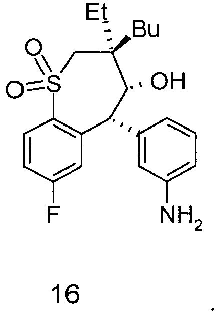

23. Verbindung der Formel 16B

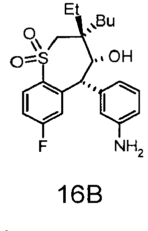

24. Verbindung der Formel 16C

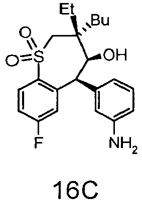

25. Verbindung der Formel 17B

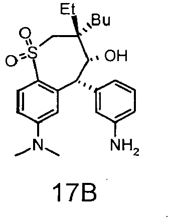

26. Verbindung der Formel 17C

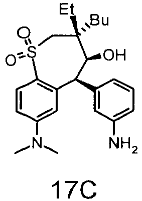

27. Verfahren zur Herstellung der Verbindung der Formel 2a 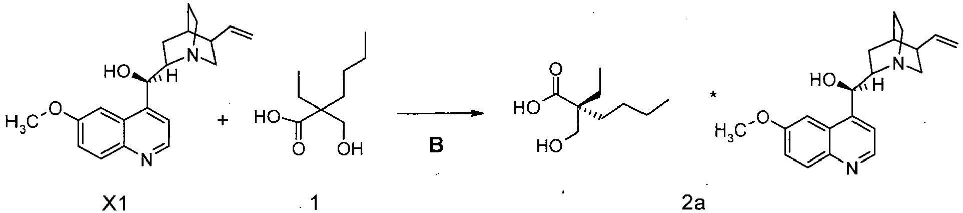 **dadurch gekennzeichnet, dass** sie aus der Verbindung der Formel X1 und der Verbindung der Formel 1 hergestellt und dann gegebenenfalls in einem geeigneten Lösungsmittel oder Lösungsmittelgemisch wie zum Beispiel Toluol, n-Butylacetat/Heptan oder Aceton/Wasser umkristallisiert wird.

28. Verfahren zur Herstellung der Verbindung der Formel 2a, gemäß Anspruch 27, **dadurch gekennzeichnet, dass** als Lösungsmittel oder Lösungsmittelgemisch zum Umkristallisieren Toluol, n-Butylacetat/Heptan oder Aceton/Wasser verwendet wird.

29. Verfahren zur Herstellung der Verbindung der Formel 2 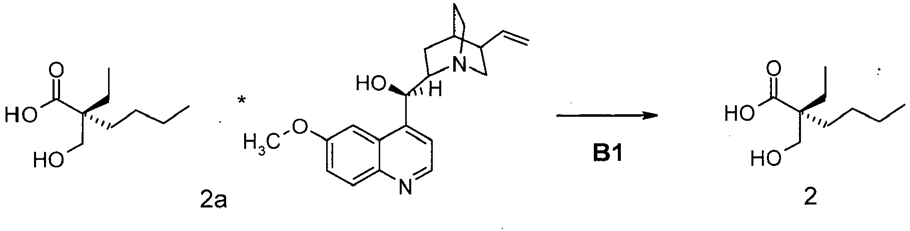 **dadurch gekennzeichnet, dass** sie aus der Verbindung der Formel 2a durch wässrige Aufarbeitung und Extraktion mit einem geeigneten Lösungsmittel gewonnen wird.

30. Verfahren zur Herstellung der Verbindung der Formel 2 gemäß Anspruch 29, **dadurch gekennzeichnet, dass** das geeignete Lösungsmittel Toluol, Ethylacetat oder Dichlormethan ist.

31. Verfahren zur Herstellung der Verbindung der Formel 10 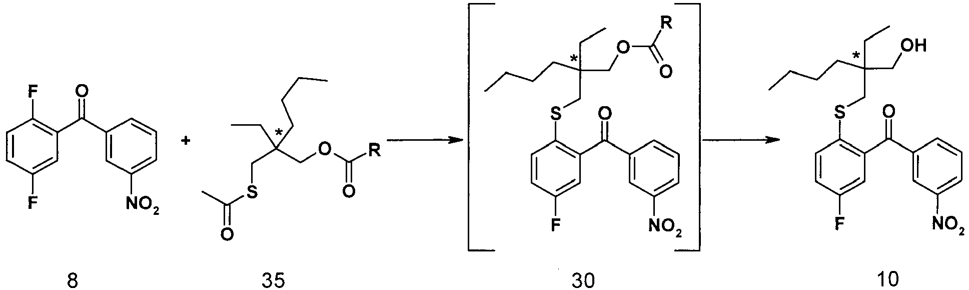 worin R in den Verbindungen der Formeln 35 und 30 die Bedeutung (C₁-C₆)-Alkyl hat, **dadurch gekennzeichnet, dass** in einem ersten Schritt
die Verbindung der Formel 8 mit der Verbindung der Formel 35a in Gegenwart einer wässrigen Base umgesetzt wird und dann in einem zweiten Schritt
das erhaltene Gemisch bestehend aus der Verbindung der Formel 30 und der Verbindung der Formel 10 durch alkalische Hydrolyse vollständig in die Verbindung der Formel 10 überführt wird.

32. Verfahren zur Herstellung der Verbindung der Formel 10 gemäß Anspruch 31, **dadurch gekennzeichnet, dass** als wässrige Base Natriumcarbonat, Kaliumcarbonat, oder Caesiumcarbonat verwendet wird.

33. Verfahren zur Herstellung der Verbindung der Formel 11 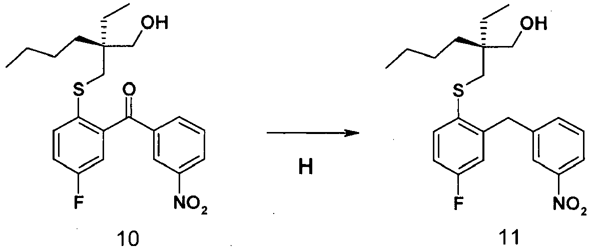 **dadurch gekennzeichnet, dass** die Verbindung der Formel 10 mit einem geeigneten Reduktionsmittel zur Verbindung der Formel 11 umgesetzt wird.

34. Verfahren zur Herstellung der Verbindung der Formel 11 gemäß Anspruch 33, **dadurch gekennzeichnet, dass** als geeignetes Reduktionsmittel Hydrophosphorigesäure/Iod, Natriumborhydrid/Aluminium-(III)-chlorid, Triethylsilan/Trifluoressigsäure, Isobutylaluminiumdichlorid, Butylsilan/Bortrifluorid, Polyhydroxymethylsilan (PHMS) oder Triethylsilan/Bortrifluorid verwendet wird.

35. Verfahren zur Herstellung der Verbindung der Formel 12 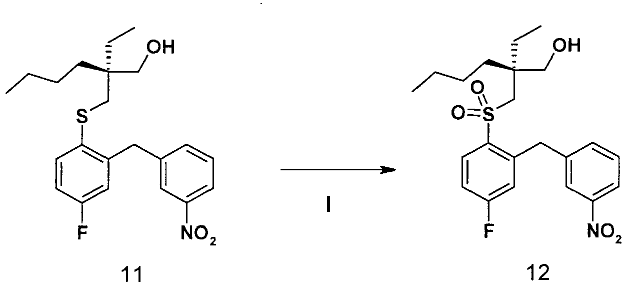 **dadurch gekennzeichnet, dass** die Verbindung der Formel 11 mit einem geeigneten Oxydationsmittel zur Verbindung der Formel 12 umgesetzt wird.

36. Verfahren zur Herstellung der Verbindung der Formel 12 gemäß Anspruch 35, **dadurch gekennzeichnet, dass** als geeignetes Oxydationsmittel Natriumperborat, Wasserstoffperoxid/Natriumwolframat, Wasserstoffperoxid/Molybdän-(IV)-oxiddichlorid, Oxone oder Wasserstoffperoxid/Acetbnitril/Ethanol verwendet wird.

37. Verfahren zur Herstellung der Verbindung der Formel 13 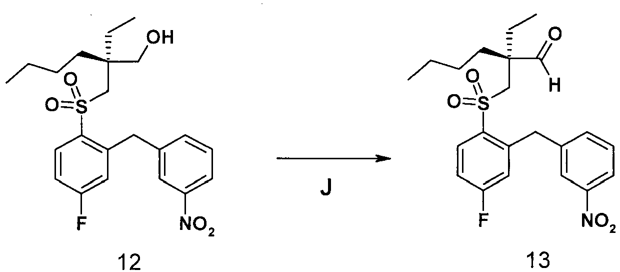 **dadurch gekennzeichnet, dass** die Verbindung der Formel 12 mit einem geeigneten Oxydationsmittel zur Verbindung der Formel 12 umgesetzt wird.

38. Verfahren zur Herstellung der Verbindung der Formel 13 gemäß Anspruch 37, **dadurch gekennzeichnet, dass** als geeignetes Oxydationsmittel Oxaylchlorid/DMSO, Schwefeltrioxid-Pyridin Komplex/DMSO, Pyridiniumdichromat, Periodan oder Natriumhypochlorid/TEMPO verwendet wird.

39. Verfahren zur Herstellung der Verbindungen der Formeln 15 und 15A 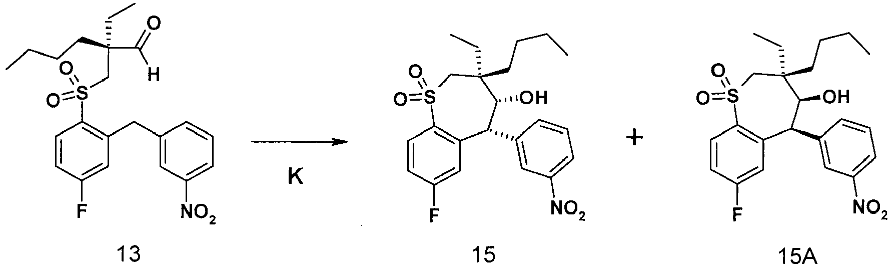 **dadurch gekennzeichnet, dass** die Verbindung der Formel 13 mit einer geeigneten Base zu den Verbindungen der Formeln 15 und 15A umgesetzt wird.

40. Verfahren zur Herstellung der Verbindungen der Formeln 15 und 15A, gemäß Anspruch 39, **dadurch gekennzeichnet, dass** als geeignete Base Natriumcarbonat, Kaliumcarbonat, Caesiumcarbonat, Natriummethylat, Kaliummethylat, Natriumethylat, Kaliumethylat, Natrium-tert.butylat oder Kalium-tert.-butylat verwendet wird.

41. Verfahren zur Herstellung der Verbindung der Formel 16 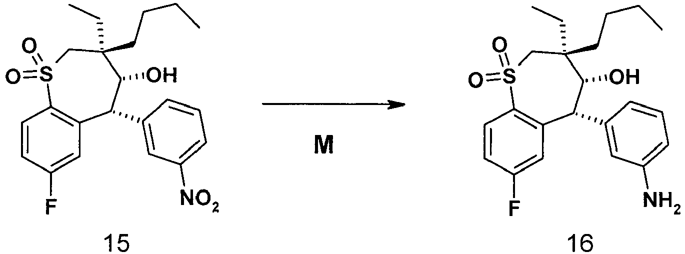 **dadurch gekennzeichnet, dass** die Verbindung der Formel 15 mit einem geeigneten Reduktionsmittel zur Verbindung der Formel 16 umgesetzt wird.

42. Verfahren zur Herstellung der Verbindung der Formel 16, gemäß Anspruch 41, **dadurch gekennzeichnet, dass** Wasserstoff/Palladium auf Aktivkohle als geeignetes Reduktionsmittel verwendet wird.

43. Verfahren zur Herstellung der Verbindung der Formel 17 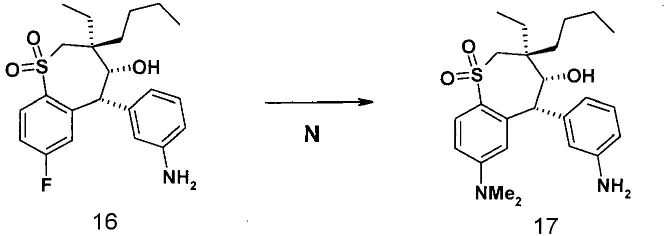 **dadurch gekennzeichnet, dass** die Verbindung der Formel 16 mit Dimethylamin zur Verbindung der Formel 17 umgesetzt wird.

44. Verfahren zur Herstellung der Verbindung der Formel 17 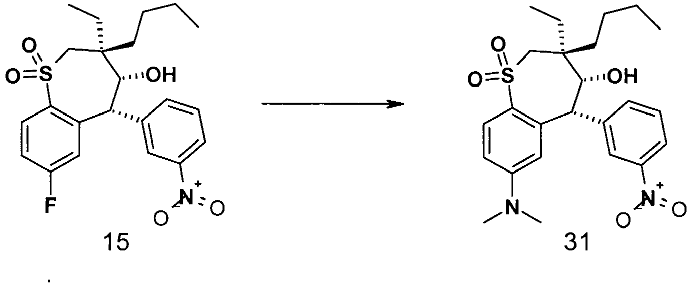 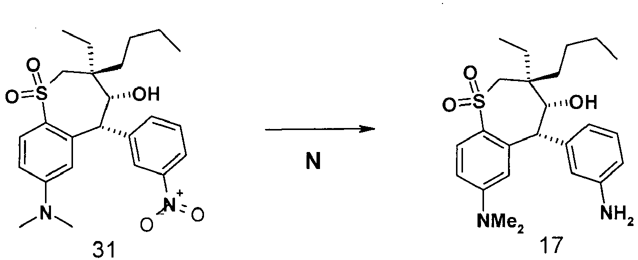 **dadurch gekennzeichnet, dass** die Verbindung der Formel 15 in einem ersten Schritt mit Dimethylamin zur Verbindung der Formel 31 umgesetzt wird und dann die erhaltene Verbindung 31 in einem weiteren Schritt mit Wasserstoff/Palladium auf Aktivkohle zur Verbindung der Formel 17 umgesetzt wird.

45. Verfahren zur Herstellung der Verbindung der Formel I, gemäß Anspruch 1, **dadurch gekennzeichnet, dass** darin bedeuten
R2, R2' R3, R3', R4, R4', R5, R5' unabhängig voneinander H, OH, -(CH₂)-OH, (C₁-C₆)-Alkylen-S(O)ₚ-R6, (C₁-C₆)-Alkylen-O-S(O)ₚ-R6, -O-(CH₂)ₘ-Phenyl, -(CH₂)-O-(CH₂)ₘ-Phenyl,
wobei immer mindestens einer der Reste R2, R2', R3, R3', R4, R4', R5, R5' die Bedeutung -O-(CH₂)ₘ-Phenyl oder -(CH₂)-O-(CH₂)ₘ-Phenyl besitzt;
R6 H, OH;
n 2, 3, 4, 5, 6;
m 1,2,3,4,5,6;
p 0, 1, 2;

46. Verfahren zur Herstellung der Verbindung der Formel I, gemäß Anspruch 1 oder 45, **dadurch gekennzeichnet, dass** darin bedeuten
R2 H;
R2' OH;
R3 -O-CH₂-Phenyl;
R3' H;
R4 H;
R4' OH;
R5 -SO₃H, -SO₃⁻NH₄⁺,
R5' H;

47. Verfahren zur Herstellung der Verbindung der Formel I, gemäß Anspruch 1, 45 oder 46, **dadurch gekennzeichnet, dass** die Verbindung der Formel I die Struktur 53 besitzt.

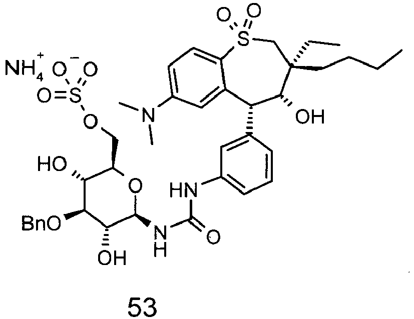

48. Verbindungen der Formeln 51a, 52a, 53a und 23a

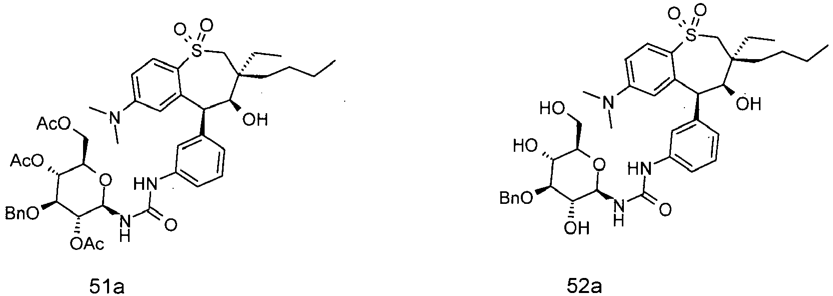

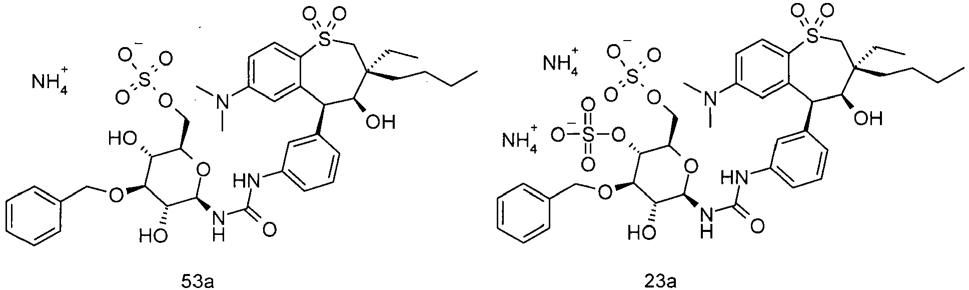

## Claims

1. A method for the production of the compound of the formula I 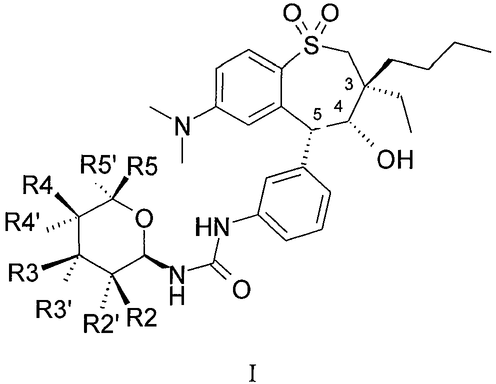 in which
R2, R2', R3, R3', R4, R4', R5, R5', independently of one another, are H, Cl, Br, I, OH, -(CH₂)-OH, CF₃, NO₂, N₃, CN, S(O)ₚ-R6, O-S(O)ₚ-R6, (C₁-C₆)-alkylene-S(O)ₚ-R6, (C₁-C₆)-alkylene-O-S(O)ₚ-R6, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where, in the alkyl radicals, one, more, or all hydrogen(s) can be replaced by fluorine;
phenyl, -(CH₂)-phenyl, -(CH₂)ₙ-phenyl, O-phenyl, O-(CH₂)ₘ-phenyl, -(CH₂)-O-(CH₂)ₘ-phenyl, where the phenyl ring may be mono- to trisubstituted with F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
where always at least one of the radicals R2, R2', R3, R3', R4, R4', R5, R5' has the meaning -O-(CH₂)ₘ-phenyl or -(CH₂)-O-(CH₂)ₘ-phenyl, where the phenyl ring may be mono- to trisubstituted with F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R6 is H, OH, (C₁-C₆)-alkyl, NH₂, NH(C₁-C₆)-alkyl, N((C₁-C₆)-alkyl)₂;
n is 2, 3, 4, 5, 6;
m is 1, 2, 3, 4, 5, 6;
p is 0, 1,2;
which comprises
G) reacting the compound of the formula 5 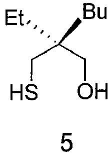 with a compound of the formula 8 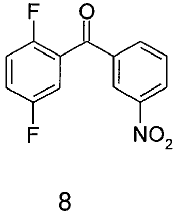 to give the compound of the formula 10 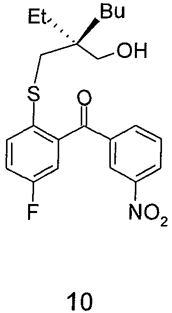 and then in a further process step
H) reacting the compound of the formula 10 in the presence of BF₃ and Et₃SiH to give a compound of the formula 11 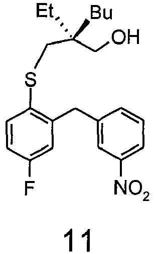 and then in a further process step
I) reacting the compound of the formula 11 in the presence of H₂O₂ to give compound 12 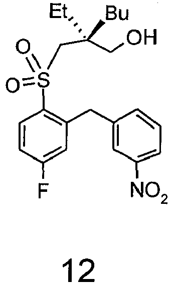 and then in a further process step
J) reacting the compound of the formula 12 in the presence of TEMPO (2,2,6,6-tetramethylpiperidinyloxyl) to give compound 13 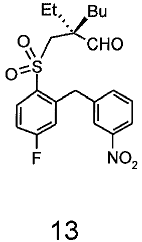 and then in a further process step
K) reacting the compound of the formula 13 in the presence of tBuOK in THF to give compound 15/15A 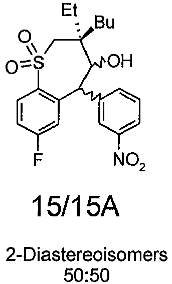 and then in a further process step
L) isolating the compound of the formula 15 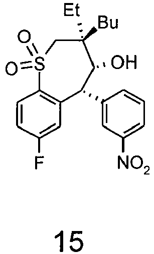 and then in a further process step
M) reacting the compound of the formula 15 with the help of H₂/Pd-C to give a compound of the formula 16 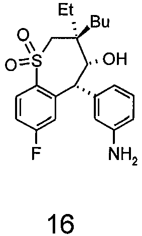 and then in a further process step
N) reacting the compound of the formula 16 in the presence of HNMe₂ to give a compound of the formula 17/17A 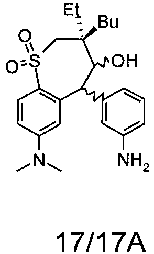 and then in a further process step
O) isolating the compound of the formula 17 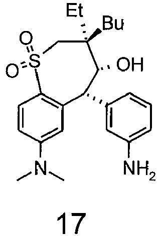 and then in a further process step
P) reacting the compound 17 with the compound 18, in which the radicals have the meanings given above 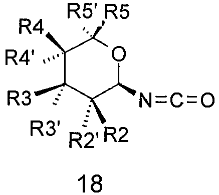 to give a compound of the formula 19 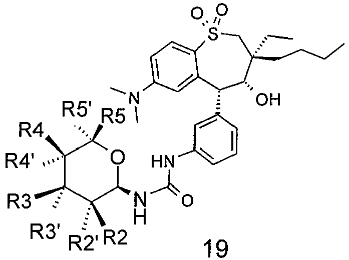 and then optionally in a further process step
Q) reacting the compound of the formula 19, in which the radicals have the meanings given above, to give a compound of the formula 20, in which the radicals have the meanings given above, 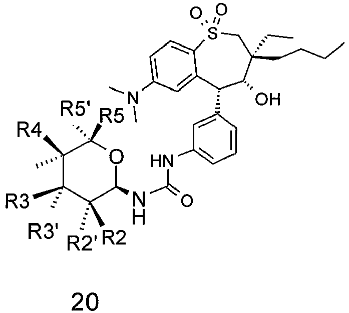 where any protective groups present are cleaved off, and then optionally in a further process step
R) reacting the compound of the formula 20, in which the radicals have the meanings given above, to give a compound of the formula Ia, in which the radicals have the meanings given above, 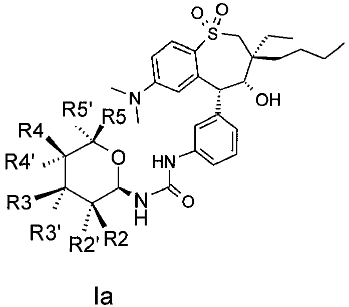 as a result of which the substituents R2, R2', R3, R3', R4, R4', R5, R5' from formula 20 can be exchanged.

2. A compound of the formula 2a

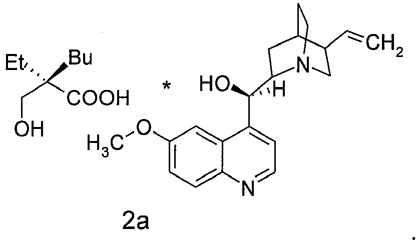

3. A compound of the formula 4

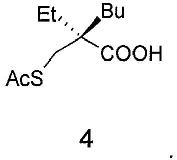

4. A compound of the formula 4a

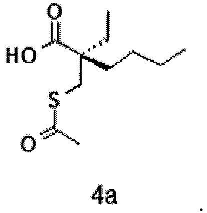

5. A compound of the formula 5

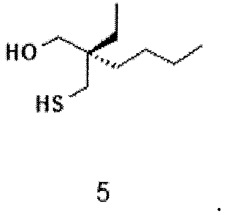

6. A compound of the formula 5a

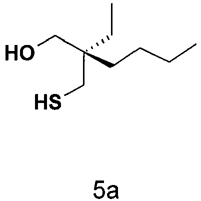

7. A racemate of the comopounds of the formula 35/35a 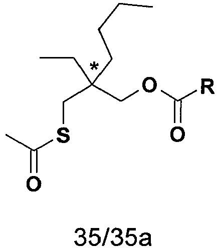 in which
R is (C₁-C₆)-alkyl.

8. A compound of the formula 35 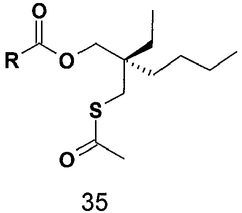 in which
R is (C₁-C₆)-alkyl.

9. A compound of the formula 35a 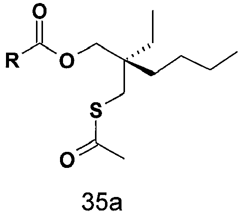 in which
R is (C₁-C₆)-alkyl.

10. A compound of the formula 8

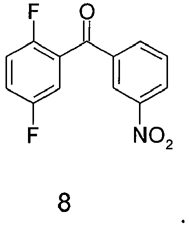

11. A compound of the formula 10

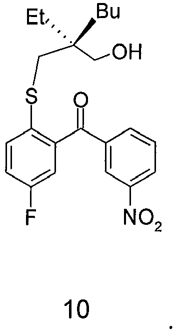

12. A compound of the formula 10a

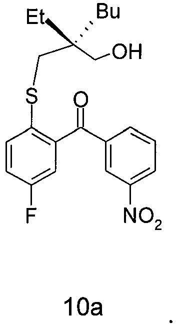

13. A compound of the formula 11

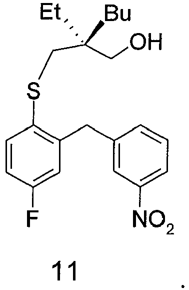

14. A compound of the formula 11a

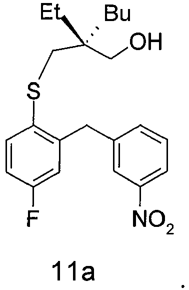

15. A compound of the formula 12

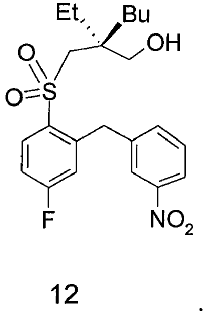

16. A compound of the formula 12a

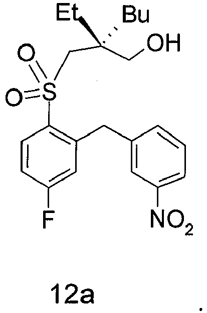

17. A compound of the formula 13

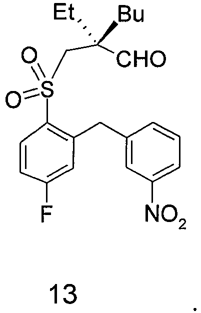

18. A compound of the formula 13a

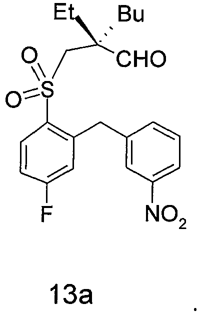

19. A compound of the formula 15

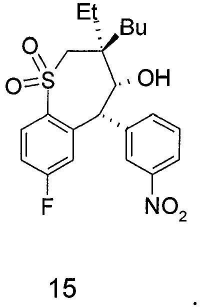

20. A compound of the formula 15B

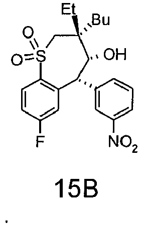

21. A compound of the formula 15C

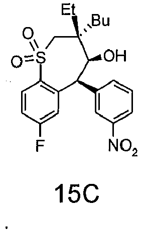

22. A compound of the formula 16

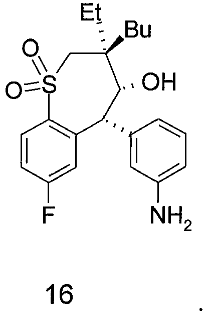

23. A compound of the formula 16B

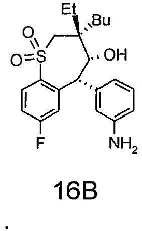

24. A compound of the formula 16C

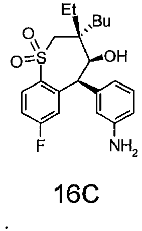

25. A compound of the formula 17B

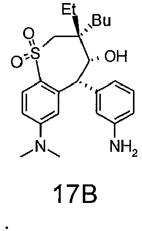

26. A compound of the formula 17C

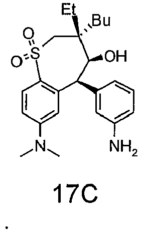

27. A method for the production of the compound of the formula 2a 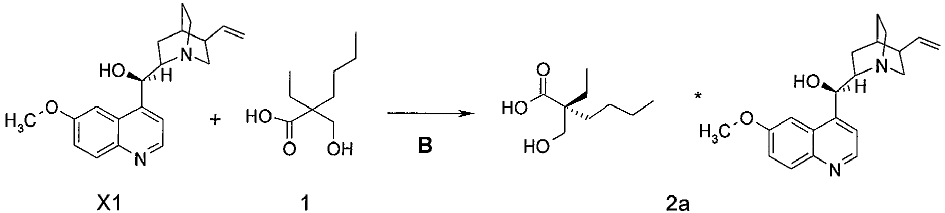 which comprises producing said compound from the compound of the formula X1 and the compound of the formula 1 and then, where appropriate, recrystallizing said compound in a suitable solvent or solvent mixture, such as, for example, toluene, n-butyl acetate/heptane or acetone/water.

28. The method for the production of the compound of the formula 2a as claimed in claim 27, wherein the solvent or solvent mixture used for the recrystallization is toluene, n-butyl acetate/heptane or acetone/water.

29. A method for the production of the compound of the formula 2 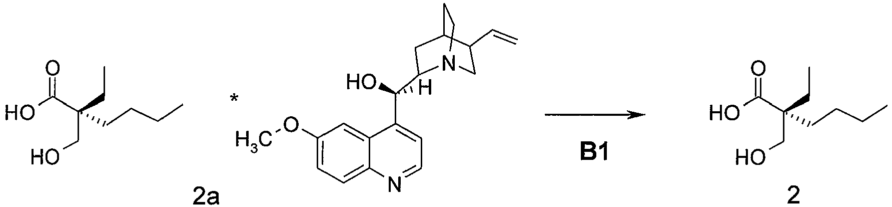 which comprises obtaining them from the compound of the formula 2a by aqueous work-up and extraction with a suitable solvent.

30. The method for the production of the compound of the formula 2 as claimed in claim 29, wherein the suitable solvent is toluene, ethyl acetate or dichloromethane.

31. A method for the production of the compound of the formula 10 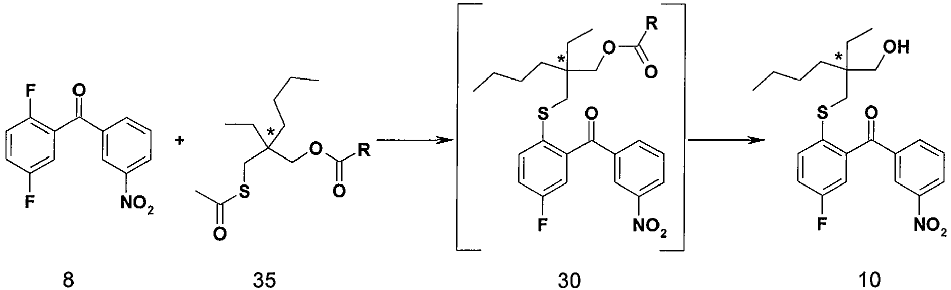 in which R in the compounds of the formulae 35 and 30 has the meaning (C₁-C₆)-alkyl, which comprises, in a first step,
reacting the compound of the formula 8 with the compound of the formula 35a in the presence of an aqueous base and then, in a second step,
completely converting the resulting mixture consisting of the compound of formula 30 and the compound of the formula 10 to the compound of formula 10 by alkaline hydrolysis.

32. The method for the production of the compound of the formula 10 as claimed in claim 31, wherein the aqueous base used is sodium carbonate, potassium carbonate or cesium carbonate.

33. A method for the production of the compound of the formula 11 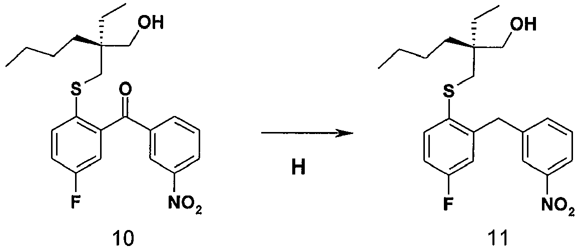 which comprises reacting the compound of the formula 10 with a suitable reducing agent to give the compound of the formula 11.

34. The method for the production of the compound of the formula 11 as claimed in claim 33, wherein hydrophosphorous acid/iodine, sodium borohydride/aluminium(III) chloride, triethylsilane/trifluoroacetic acid, isobutylaluminum dichloride, butylsilane/boron trifluoride, polyhydroxymethylsilane (PHMS) or triethylsilane/boron trifluoride is used as a suitable reducing agent.

35. A method for the production of the compound of the formula 12 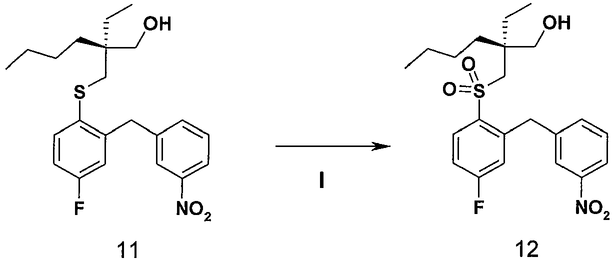 which comprises reacting the compound of the formula 11 with a suitable oxidizing agent to give the compound of the formula 12.

36. The method for the production of the compound of the formula 12 as claimed in claim 35, wherein sodium perborate, hydrogen peroxide/sodium tungstate, hydrogen peroxide/molybdenum(IV) oxide dichloride, oxones or hydrogen peroxide/acetonitrile/ethanol is used as suitable oxidizing agent.

37. The method for the production of the compound of the formula 13 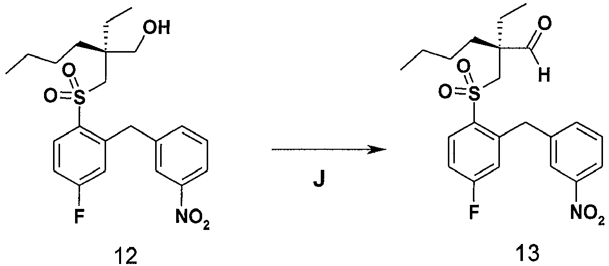 which comprises reacting the compound of the formula 12 with a suitable oxidizing agent to give the compound of the formula 12.

38. The method for the production of the compound of the formula 13 as claimed in claim 37, wherein oxayl chloride/DMSO, sulfur trioxide-pyridine complex/DMSO, pyridinium dichromate, periodane or sodium hypochloride/TEMPO is used as oxidizing agent.

39. A method for the production of the compounds of the formulae 15 and 15A 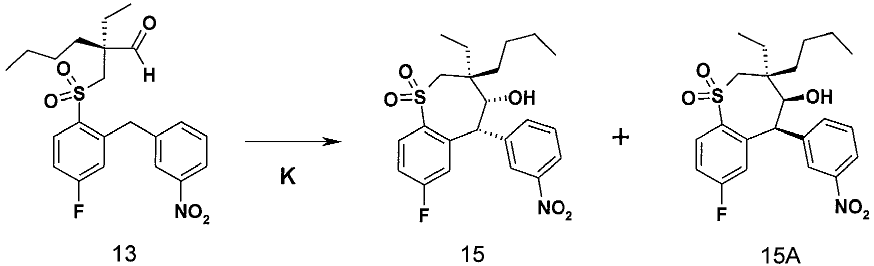 which comprises reacting the compound of the formula 13 with a suitable base to give the compounds of the formulae 15 and 15A.

40. The method for the production of the compounds of the formulae 15 and 15A, as claimed in claim 39, wherein sodium carbonate, potassium carbonate, cesium carbonate, sodium methylate, potassium methylate, sodium ethylate, potassium ethylate, sodium tert-butylate or potassium tert-butylate is used as suitable base.

41. A method for the production of the compound of the formula 16 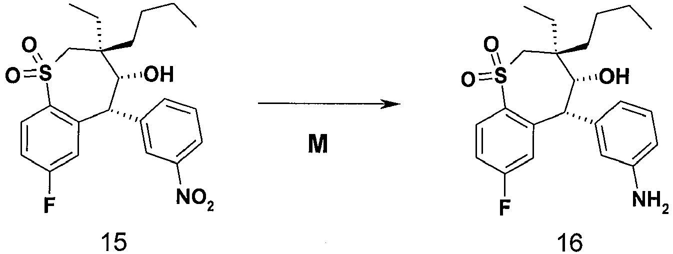 which comprises reacting the compound of the formula 15 with a suitable reducing agent to give the compound of the formula 16.

42. The method for the production of the compound of the formula 16 as claimed in claim 41, wherein hydrogen/palladium on activated carbon is used as suitable reducing agent.

43. A method for the production of the compound of the formula 17 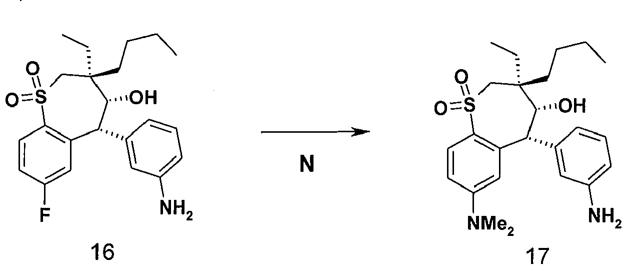 which comprises reacting the compound of the formula 16 with dimethylamine to give the compound of the formula 17.

44. A method for the production of the compound of the formula 17 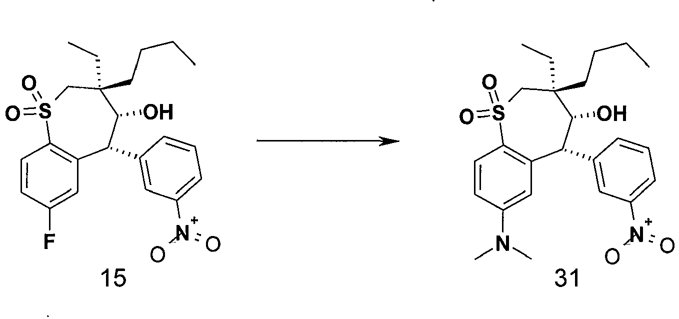 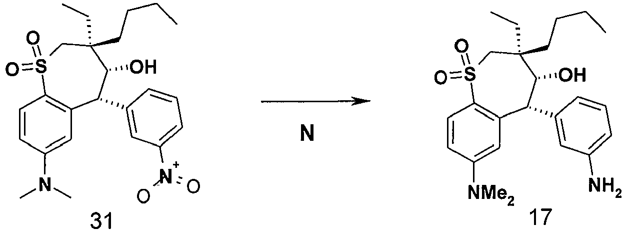 which comprises reacting the compound of the formula 15 in a first step with dimethylamine to give the compound of the formula 31 and then reacting the resulting compound 31 in a further step with hydrogen/palladium on activated carbon to give the compound of the formula 17.

45. The method for the production of the compound of the formula I as claimed in claim 1, wherein
R2, R2' R3, R3', R4, R4', R5, R5', independently of one another, are H, OH, -(CH₂)-OH, (C₁-C₆)-alkylene-S(O)ₚ-R6, (C₁-C₆)-alkylene-O-S(O)ₚ-R6, -O-(CH₂)ₘ-phenyl, -(CH₂)-O-(CH₂)ₘ-phenyl,
where always at least one of the radicals R2, R2', R3, R3', R4, R4', R5, R5' has the meaning -O-(CH₂)ₘ-phenyl or -(CH₂)-O-(CH₂)ₘ-phenyl;
R6 is H, OH;
n is 2, 3, 4, 5, 6;
m is 1, 2, 3, 4, 5, 6;
p is 0, 1,2.

46. The method for the production of the compound of the formula I as claimed in claim 1 or 45, wherein
R2 is H;
R2' is OH;
R3 is -O-CH₂-phenyl;
R3' is H;
R4 is H;
R4' is OH;
R5 is -SO₃H, -SO₃⁻NH₄⁺,
R5' is H;

47. A method for the production of the compound of the formula I as claimed in claim 1,45 or 46, wherein the compound of the formula I has the structure 53.

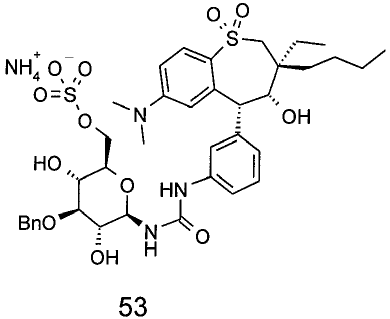

48. The compound of the formula 51a, 52a, 53a and 23a

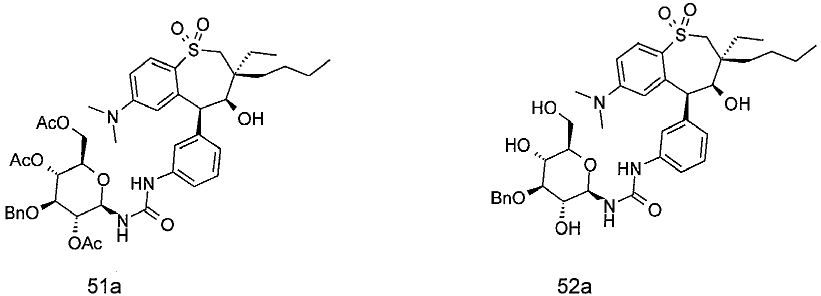

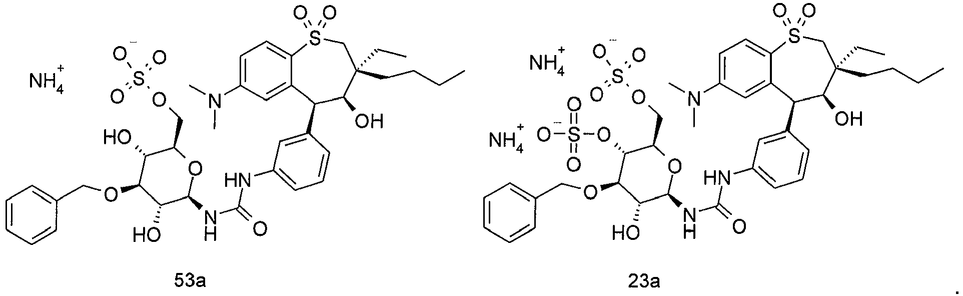

## Revendications

1. Procédé pour la préparation du composé de formule I 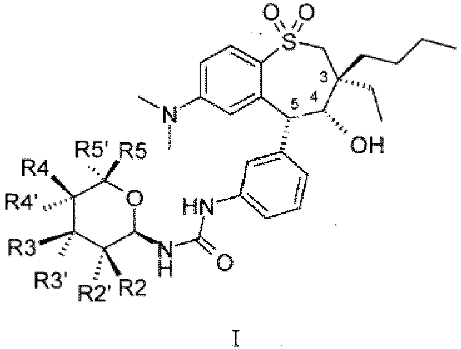 dans laquelle
R2, R2', R3, R3', R4, R4', R5, R5' représentent indépendamment les uns des autres H, Cl, Br, I, OH, -(CH₂)-OH, CF₃, NO₂, N₃, CN, S(O)p-R6, O-S(O)ₚ-R6, un groupe alkylène (C₁-C₆) -S(O)ₚ-R6, alkylène (C₁-C₆)-O-S(O)ₚ-R6, COOH, COO-alkyle (C₁-C₆), CONH₂, CONH-alkyle (C₁-C₆), CON[alkyle (C₁-C₆)]₂, alkyle en C₁-C₆, alcényle en C₂-C₆, alcynyle en C₂-C₆, O-alkyle(C₁-C₆), dans les radicaux alkyle un, plusieurs ou tous les atome(s) d'hydrogène pouvant être remplacé(s) par un/des atome(s) de fluor ;
un groupe phényle, -(CH₂)-phényle, -(CH₂)ₙ-phényle, O-phényle, O-(CH₂)ₘ-phényle, -(CH₂)-O-(CH₂)ₘ-phényle, le cycle phényle pouvant être une à trois fois substitué par F, Cl, Br, I, OH, CF₃, NO₂, CN, OCF₃, O-alkyle (C₁-C₆), alkyle en C₁-C₆, NH₂, NH-alkyle (C₁-C₆), N (alkyle (C₁-C₆))₂, SO₂-CH₃, COOH, COO-alkyle(C₁-C₆), CONH₂ ;
au moins un des radicaux R2, R2', R3, R3', R4, R4', R5, R5' ayant toujours la signification d'un groupe -O-(CH₂)ₘ-phényle ou -(CH₂)-O-(CH₂)ₘ-phényle, le cycle phényle pouvant être une à trois fois substitué par F, Cl, Br, I, OH, CF₃, NO₂, CN, OCF₃, O-alkyle (C₁-C₆), alkyle en C₁-C₆, NH₂, NH-alkyle (C₁-C₆), N(alkyle (C₁-C₆))₂, SO₂-CH₃, COOH, COO-alkyle (C₁-C₆), CONH₂ ;
R6 représente H, OH, un groupe alkyle en C₁-C₆, NH₂, NH-alkyle (C₁-C₆), N(alkyle(C₁-C₆))₂ ;
n vaut 2, 3, 4, 5, 6 ;
m vaut 1, 2, 3, 4, 5, 6 ;
p vaut 0, 1, 2 ;
**caractérisé en ce que**
G) on fait réagir un composé de formule 5 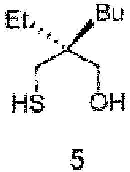 avec un composé de formule 8 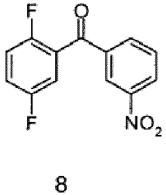 pour aboutir au composé de formule 10 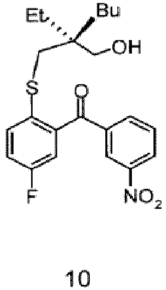 et ensuite, dans une nouvelle étape de processus
H) on convertit le composé de formule 10, en présence de BF₃ et Et₃SiH, en un composé de formule 11 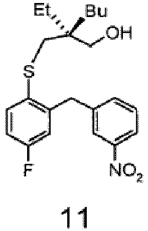 et ensuite, dans une nouvelle étape de processus
I) on convertit le composé de formule II, en présence de H₂O₂, en le composé 12 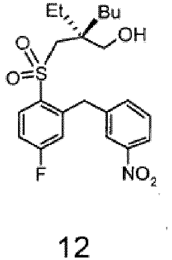 et ensuite, dans une nouvelle étape de processus
J) on convertit le composé de formule 12, en présence de TEMPO (2,2,6,6-tétraméthylpipéridinyl-oxyle), en le composé 13 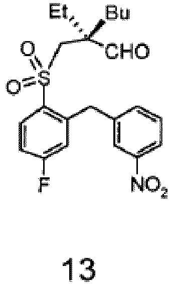 et ensuite, dans une nouvelle étape de processus
K) on convertit le composé de formule 13, en présence de tBuOK dans du THF, en le composé 15/15A 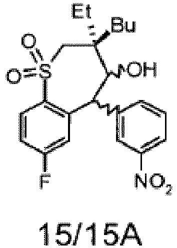 2 diastéréoisomères 50:50
et ensuite, dans une nouvelle étape de processus
L) on isole le composé de formule 15 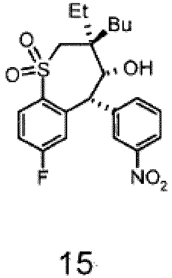 et ensuite, dans une nouvelle étape de processus
M) on convertit le composé de formule 15, à l'aide de H₂/Pd-C, en un composé de formule 16 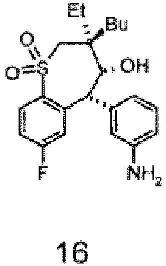 et ensuite, dans une nouvelle étape de processus
N) on convertit le composé de formule 16, en présence de HNMe₂, en un composé de formule 17/17A 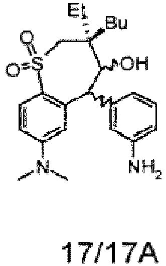 et ensuite, dans une nouvelle étape de processus
O) on isole le composé de formule 17 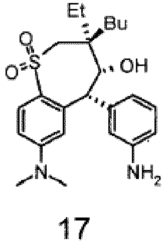 et ensuite, dans une nouvelle étape de processus
P) on fait réagir le composé 17 avec le composé 18, dans lequel les radicaux ont les significations indiquées plus haut 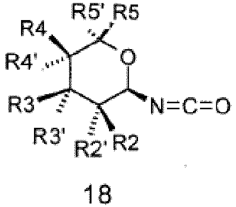 pour aboutir à un composé de formule 19 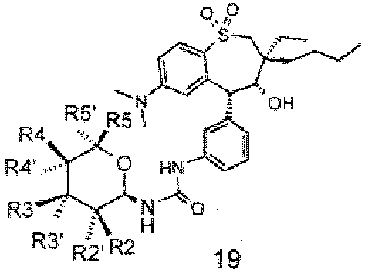 et ensuite, en option, dans une nouvelle étape de processus
Q) on convertit le composé de formule 19, dans laquelle les radicaux ont les significations indiquées plus haut, en un composé de formule 20, dans laquelle les radicaux ont les significations indiquées plus haut, 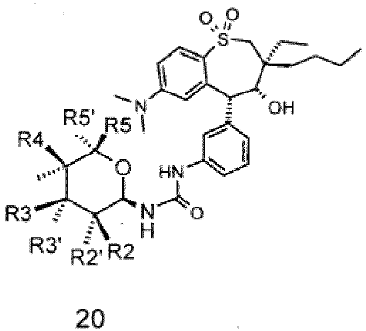 en éliminant les groupes protecteurs éventuellement présents, et ensuite, en option, dans une nouvelle étape de processus
R) on convertit le composé de formule 20, dans laquelle les radicaux ont les significations indiquées plus haut, en un composé de formule Ia, dans laquelle les radicaux ont les significations indiquées plus haut, 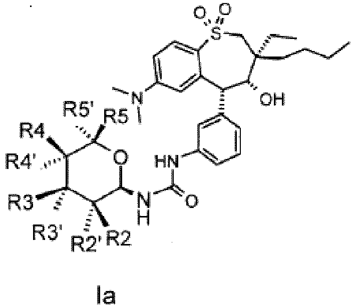 ce par quoi les substituants R2, R2', R3, R3', R4, R4', R5, R5' de la formule 20 peuvent être échangés.

2. Composé de formule 2a

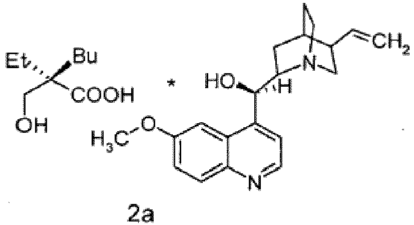

3. Composé de formule 4

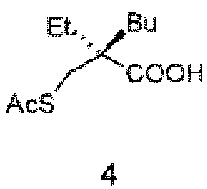

4. Composé de formule 4a

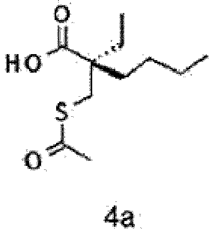

5. Composé de formule 5

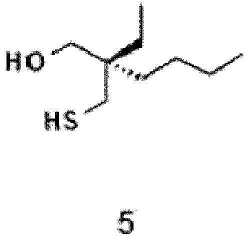

6. Composé de formule 5a

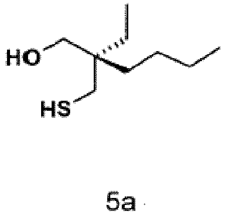

7. Racémate des composés de formule 35/35a 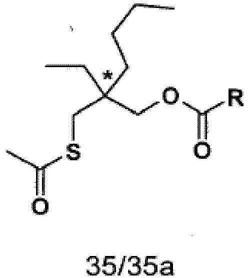 dans laquelle
R représente un groupe alkyle en C₁-C₆.

8. Composé de formule 35 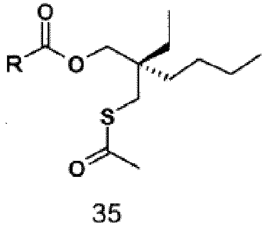 dans laquelle
R représente un groupe alkyle en C₁-C₆.

9. Composé de formule 35a 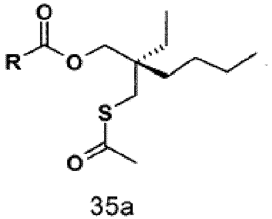 dans laquelle
R représente un groupe alkyle en C₁-C₆.

10. Composé de formule 8

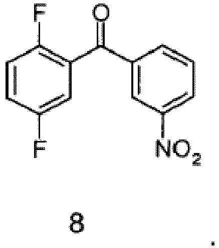

11. Composé de formule 10

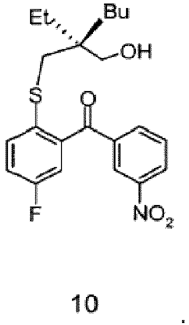

12. Composé de formule 10a

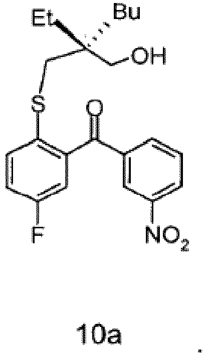

13. Composé de formule 11

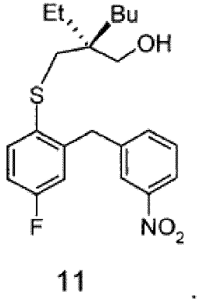

14. Composé de formule 11a

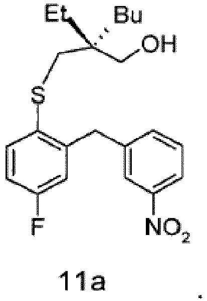

15. Composé de formule 12

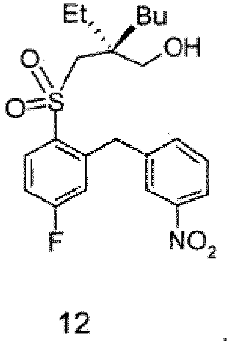

16. Composé de formule 12a

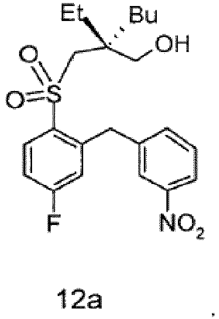

17. Composé de formule 13

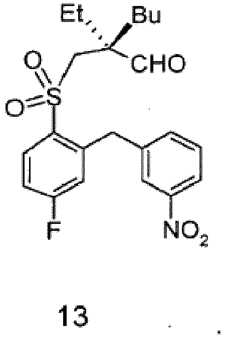

18. Composé de formule 13a

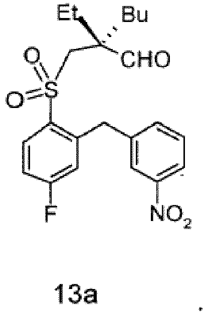

19. Composé de formule 15

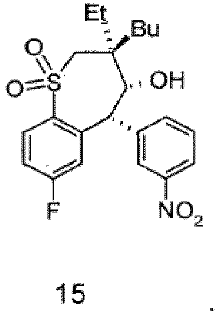

20. Composé de formule 15B

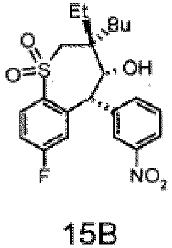

21. Composé de formule 15C

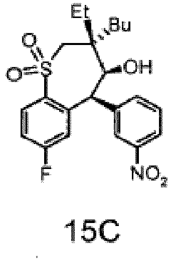

22. Composé de formule 16

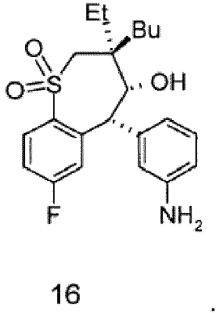

23. Composé de formule 16B

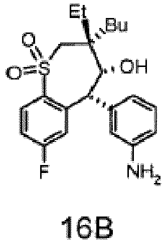

24. Composé de formule 16C

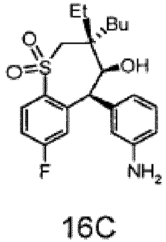

25. Composé de formule 17B

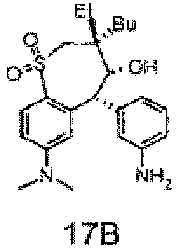

26. Composé de formule 17C

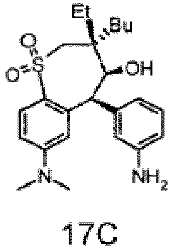

27. Procédé pour la préparation du composé de formule 2a 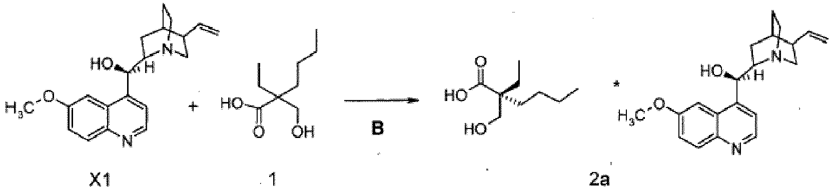 **caractérisé en ce qu'**on le prépare à partir du composé de formule X1 et du composé de formule 1 et ensuite éventuellement on le fait recristalliser dans un solvant ou mélange de solvants convenable comme par exemple le toluène, un mélange d'acétate de n-butyle/heptane ou d'acétone/eau.

28. Procédé pour la préparation du composé de formule 2a, selon la revendication 27, **caractérisé en ce que** comme solvant ou mélange de solvants pour la recristallisation on utilise le toluène, un mélange d'acétate de n-butyle/heptane ou d'acétone/eau.

29. Procédé pour la préparation du composé de formule 2 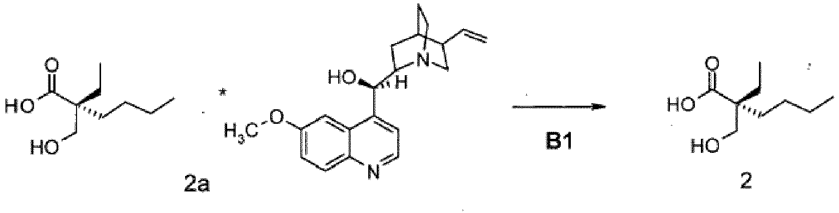 **caractérisé en ce qu'**on l'obtient à partir du composé de formule 2a par traitement final aqueux et extraction à l'aide d'un solvant approprié.

30. Procédé pour la préparation du composé de formule 2 selon la revendication 29, **caractérisé en ce que** le solvant approprié est le toluène, l'acétate d'éthyle ou le dichlorométhane.

31. Procédé pour la préparation du composé de formule 10 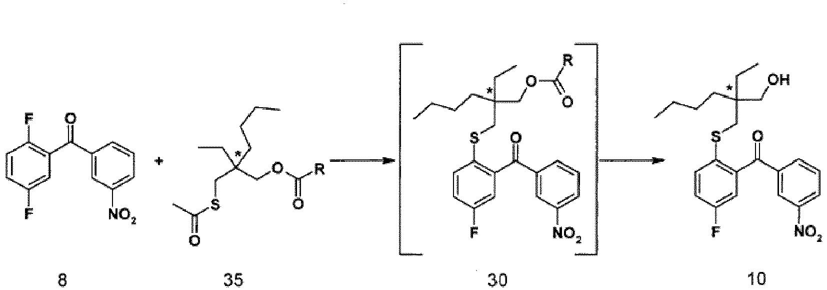 dans laquelle R a dans les composés de formules 35 et 30 la signification d'un groupe alkyle en C₁-C₆, **caractérisé en ce que** dans une première étape
on fait réagir le composé de formule 8 avec le composé de formule 35a en présence d'une base aqueuse, et ensuite dans une seconde étape
on convertit totalement par hydrolyse alcaline le mélange obtenu, constitué du composé de formule 30 et du composé de formule 10, en le composé de formule 10.

32. Procédé pour la préparation du composé de formule 10 selon la revendication 31, **caractérisé en ce qu'**on utilise comme base aqueuse le carbonate de sodium, le carbonate de potassium ou le carbonate de césium.

33. Procédé pour la préparation du composé de formule 11 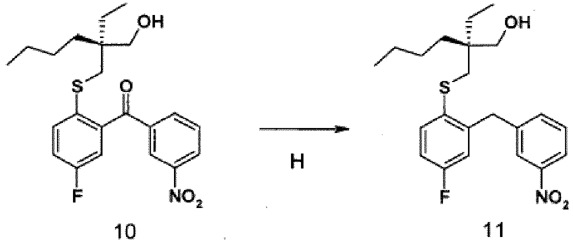 **caractérisé en ce qu'**on convertit le composé de formule 10, à l'aide d'un réducteur approprié, en le composé de formule 11.

34. Procédé pour la préparation du composé de formule 11 selon la revendication 33, **caractérisé en ce qu'**on utilise comme réducteur approprié l'acide hydrophosphoreux/iode, le borohydrure de sodium/chlorure d'aluminium-(III), le triéthylsilane/ acide trifluoroacétique, le dichlorure d'isobutylaluminium, le butylsilane/trifluorure de bore, le polyhydroxyméthylsilane (PHMS) ou le triéthylsilane/trifluorure de bore.

35. Procédé pour la préparation du composé de formule 12 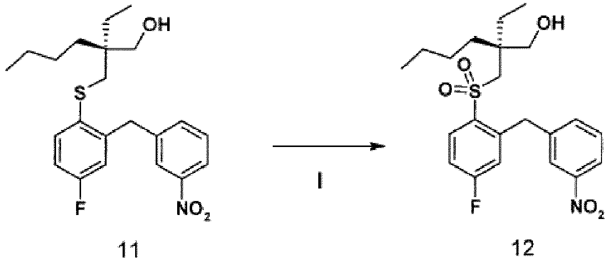 **caractérisé en ce qu'**on convertit le composé de formule 11, à l'aide d'un oxydant approprié, en le composé de formule 12.

36. Procédé pour la préparation du composé de formule 12 selon la revendication 35, **caractérisé en ce qu'**on utilise comme oxydant approprié le perborate de sodium, le peroxyde d'hydrogène/tungstate de sodium, le peroxyde d'hydrogène/oxydichlorure de molybdène-(IV), l'Oxone ou le peroxyde d'hydrogène/acétonitrile/ éthanol.

37. Procédé pour la préparation du composé de formule 13 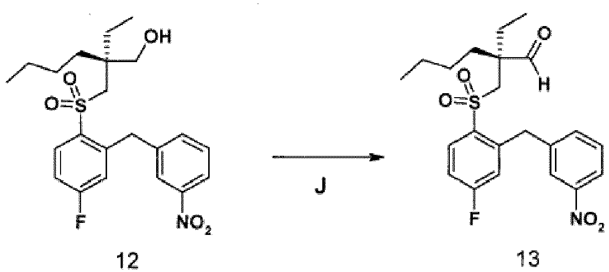 **caractérisé en ce qu'**on convertit le composé de formule 12, à l'aide d'un oxydant approprié, en le composé de formule 12.

38. Procédé pour la préparation du composé de formule 13 selon la revendication 37, **caractérisé en ce qu'**on utilise comme oxydant approprié le chlorure d'oxalyle/DMSO, le complexe trioxyde de soufre-pyridine/DMSO, le dichromate de pyridinium, un periodane ou l'hypochlorure de sodium/TEMPO.

39. Procédé pour la préparation des composés de formules 15 et 15A 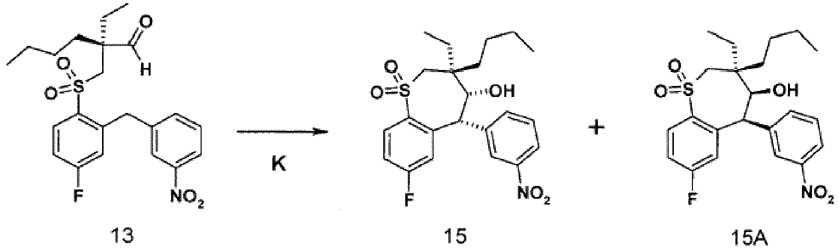 **caractérisé en ce qu'**on convertit le composé de formule 13, à l'aide d'une base appropriée, en les composés de formules 15 et 15A.

40. Procédé pour la préparation des composés de formules 15 et 15A, selon la revendication 39, **caractérisé en ce qu'**on utilise comme base appropriée le carbonate de sodium, le carbonate de potassium, le carbonate de césium, le méthylate de sodium, le méthylate de potassium, l'éthylate de sodium, l'éthylate de potassium, le tert-butylate de sodium ou le tert-butylate de potassium.

41. Procédé pour la préparation du composé de formule 16 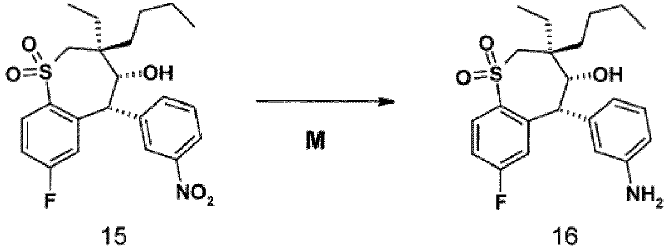 **caractérisé en ce qu'**on convertit le composé de formule 15, à l'aide d'un réducteur approprié, en le composé de formule 16.

42. Procédé pour la préparation du composé de formule 16, selon la revendication 41, **caractérisé en ce qu'**on utilise comme réducteur approprié l'hydrogène/ palladium sur charbon actif.

43. Procédé pour la préparation du composé de formule 17 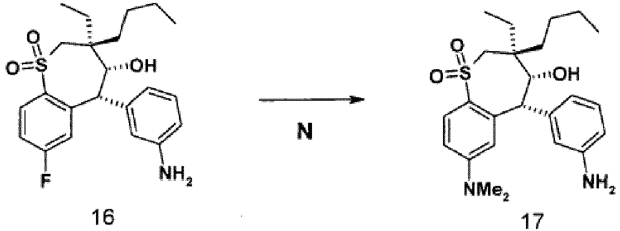 **caractérisé en ce qu'**on fait réagir le composé de formule 16 avec de la diméthylamine pour aboutir au composé de formule 17.

44. Procédé pour la préparation du composé de formule 17 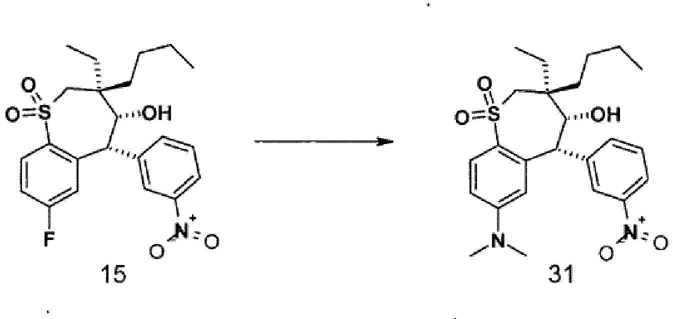 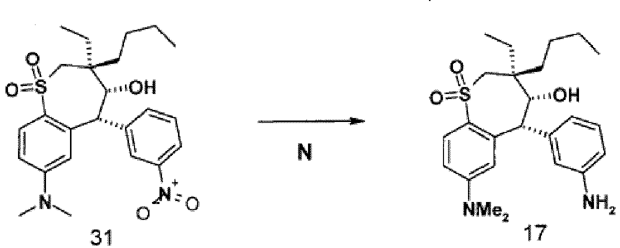 caractérisé en ce dans une première étape on fait réagir le composé de formule 15 avec de la diméthylamine, pour aboutir au composé de formule 31, et ensuite dans une nouvelle étape on convertit le composé 31 obtenu, à l'aide d'hydrogène/palladium sur charbon actif, en le composé de formule 17.

45. Procédé pour la préparation du composé de formule I, selon la revendication 1, **caractérisé en ce que** dans cette formule
R2, R2', R3, R3', R4, R4', R5, R5' représentent indépendamment les uns des autres H, OH, -(CH₂)-OH, un groupe alkylène(C₁-C₆)-S(O)ₚ-R6, alkylène (C₁-C₆) -O-S(O)ₚ-R6, O-(CH₂)ₘ-phényle, -(CH₂)-O-(CH₂)ₘ-phényle,
au moins un des radicaux R2, R2', R3, R3', R4, R4', R5, R5' ayant toujours la signification d'un groupe -O-(CH₂)ₘ-phényle ou -((CH₂)-O-(CH₂)ₘ-phényle ;
R6 représente H, OH ;
n vaut 2, 3, 4, 5, 6 ;
m vaut 1, 2, 3, 4, 5, 6 ;
p vaut 0, 1, 2.

46. Procédé pour la préparation du composé de formule I, selon la revendication 1 ou 45, **caractérisé en ce que** dans cette formule
R2 représente H ;
R2' représente OH ;
R3 représente un groupe -O-CH₂-phényle ;
R3' représente H ;
R4 représente H ;
R4' représente OH ;
R5 représente -SO₃H, -SO₃⁻NH₄⁺ ;
R5' représente H.

47. Procédé pour la préparation du composé de formule I, selon la revendication 1, 45 ou 46, **caractérisé en ce que** le composé de formule I présente la structure 53

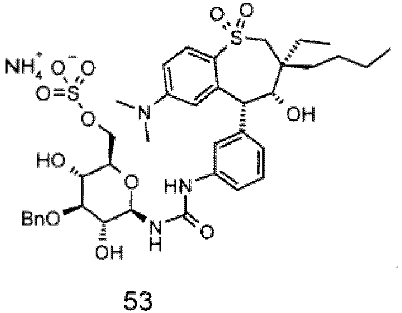

48. Composés de formules 51a, 52a, 53a et 23a
